# EUROPEAN PATENT SPECIFICATION

(11) **EP 3 188 676 B1**
(45) Date of publication and mention of the grant of the patent: **07.08.2019**
(21) Application number: 15838477.6
(22) Date of filing: 07.09.2015
(51) Int. Cl.: A61B 17/56, A61B 17/68, A61F 2/02, A61L 27/40, A61F 2/30, A61B 17/16, A61L 27/36, A61L 27/50, A61L 31/06, A61B 17/80, A61B 17/86, A61B 17/00

(54) **BIOCOMPOSITE ORTHOPEDIC IMPLANT INTRODUCER ASSEMBLY**
EINFÜHRERANDORDNUNG FÜR ORTHOPÄDISCHES BIOKOMPOSITIMPLANTAT
ENSEMBLE INTRODUCTEUR D'IMPLANT ORTHOPÉDIQUE BIOCOMPOSITE

(30) Priority: 07.09.2014 US 201462047023 P
(43) Date of publication of application: 12.07.2017
(73) Proprietor: Ossio Ltd., 3052640 Binyamina (IL)
(72) Inventor: PREISS-BLOOM, Orahn, 30900 Zichron Yaakov (IL); LINDNER, Taly Pnina, 56905 Savyon(G.Y) (IL)
(74) Representative: FRKelly
(86) International application number: PCT/IL2015/050904
(87) International publication number: WO 2016/035089

(56) References cited:
- EP-A1- 2 292 166
- EP-A2- 1 716 874
- WO-A1-2010/122019
- WO-A2-2005/077039
- US-A1- 2007 185 568
- US-A1- 2007 185 568
- US-A1- 2012 265 206
- US-A1- 2013 317 555
- ARTHREX INC.: 'BioComposite Interference Screws - A Stronger turn in ACL/PCL Reconstruction' SCIENTIFIC RESEARCH AND DEVELOPMENT 2005, XP055416379 Retrieved from the Internet: <URL:http://www,arthrex.com/knee/biocomposi te-interference-screws>

## Description

### FIELD OF THE INVENTION

The present disclosure contemplates introducer instrumentations for achieving bone and soft tissue fixation with a new class of reinforced composite biomaterials, or more particularly for percutaneously introducing of such absorbable implants into a patient.

### BACKGROUND

A new class of reinforced composite biomaterials has been recently introduced wherein a bioabsorbable and biocompatible polymer is reinforced by bioabsorbable, biocompatible glass fibers. These materials can achieve mechanical properties far exceeding the previously strongest absorbable polymers, up to even 10x flexural modulus and 5x flexural strength. Importantly, these materials also involve a compatibilizer to bind the polymer to the reinforcing fibers. Examples of such materials are described in the following two patent applications which are hereby incorporated by reference in their entirety:
1. Biocompatible composite and its use (WO2010122098)
2. Resorbable and biocompatible fibre glass compositions and their uses (WO2010122019)

For use in medical implants, these materials represent a significant benefit over metal or other permanent implants (including polymer and reinforced polymer or composite implants) in that they are absorbable and thus the implant will degrade in the body following implantation. They also represent a significant benefit over prior absorbable implants since they are much stronger and stiffer than non-reinforced absorbable polymer implants. In fact, these reinforced composite polymer materials can even approach the strength and stiffness of cortical bone, making them the first absorbable materials for use in load bearing orthopedic implant applications.

Nonetheless, while the superior mechanical properties of these absorbable, reinforced composite materials has been demonstrated in parts having simple geometries, medical implants using these materials cannot be designed according to existing implant designs due to the limitations associated with producing parts from these composite materials. Nevertheless due to these superior mechanical properties, smaller profiled implants can be designed using smaller amounts of implantable material decreasing the damage to tissue during and after surgery.

However, these mechanically superior materials lack the hardness to penetrate and cut though cortical bone on their own, although they are strong enough to aid in the implant insertion.

Several attempts have been made to resolve the above issues. However these disclosures include several drawbacks including: no option to remove the sharp bone penetrating element; a large number of steps required in the process of inserting the implant; creating a hole bigger than the implant size or as big as the implant and therefore compromising fixation.

US patent 5,522,817 describes an absorbable surgical fastener with bone penetrating elements. This assembly combines an absorbable and non-absorbable material to create an assembly that can penetrate bone and insert the implant into the desired location. However the two materials are bonded and cannot be retracted, thus leaving non-absorbable sharp parts inside the body that can potentially migrate and cause unwanted damage when the implant absorbs.

US patent 6,916,321 describes a self-tapping resorbable two-piece bone screw, comprising a cutting and threading tip fabricated from a material with strength and hardness at least equivalent to that of bone and an absorbable screw. In this case the hard non-absorbable tip enables the introduction of the implant. However, this sharp tip is not removable, again leaving behind a non-absorbable part that can migrate inside the body.

US patent 6,471,707 describes a bone screw having a bioresorbable proximal shaft portion. As with the above cited prior art, a metal portion remains in the distal side of the implant and cannot be removed. US patent application 2005/0216016 teaches a system that increase the insertion torque tolerance of a polymer based orthopedic screw with a special heat treatment for both screw and driver. This may potentially reduce failure during insertion, but does not decrease the number of steps or operation time required as this still relies on a prior step to tap holes in the injured bone prior to fixation.

It would therefore be advantageous to combine the mechanical strength of biocomposite implants with a mechanism for implantation and the ability to completely remove the introducer after insertion.

WO2005077039 (A2) discloses orthopedic implants which are at least partially absorbable. The implants of the invention may include a biocompatible material in the form of a ring, which may be used in combination with a second, more porous, absorbable material. This second material may be a continuous body or discontinuous. The implant may also include a first material connected to a full or partial wedge of a second material, the wedge being connected to the inner surface of the more dense material. Suitable materials for the first and second materials include, but are not limited to, resorbable polymer composites. The implants of the invention may also include plates for anchoring of the implant.

### SUMMARY OF THE INVENTION

The present invention overcomes the deficiencies of the background art by providing an introducer assembly for inserting reinforced biocomposite biodegradable implants into bone and soft tissue.

The introducer assembly comprises an introducer and the biocomposite implant enabling implant insertion as part of a single step or in fewer steps than required in prior art methods. The unique material strength of the biocomposite materials enables insertion techniques which are much more natural to those skilled in the art and more user-friendly then those of prior art absorbable implants lacking the biocomposite strength.

The introducer comprises metal or other material, the distal end of which is optionally in the form of a sharp drill tip, which penetrates the bone and creates a hole for the insertion of the biocomposite implant. Alternatively, the introducer penetrates the bone and creates an initial hole and then relies on the biocomposite implant to enlarge the hole. After insertion, the introducer is separated from the implant leaving the implant in the desired location.

According to some embodiments of the present invention, an introducer assembly for introducing an orthopedic implant into a subject comprises: an introducer; and the implant, wherein the implant comprises a biocomposite material; wherein the introducer initiates a cavity for the implant, wherein the assembly is inserted into the cavity to thereby extend the cavity, and wherein the introducer is retracted from the assembly leaving the implant in the cavity.

Preferably, the structural strength of the biocomposite material contributes to the structural strength of the assembly. Preferably, the implant extends the cavity. Optionally, the assembly further comprises a removable introducer sleeve and a handle. Preferably, initiating the cavity comprises penetration of bone and/or soft tissue.

Preferably, the relationship of the diameter of the introducer and the outer diameter of the implant is selected from the group consisting of: the diameter of the introducer is less than 80% of the outer diameter of the implant; the diameter of the introducer is less than 60% of the outer diameter of the implant; and the diameter of the introducer is smaller than the outer diameter of the implant. Preferably, the introducer comprises a material selected from the group consisting of: 316 L stainless steel; 316 LVM stainless steel; 304 stainless steel; titanium; nitinol; a biocompatible metal; ceramic; and a combination of the above.

Preferably, the introducer protrudes from the distal end of the assembly and is of a length selected from the group consisting of: 2-30 em; 5-20 em; and 8-15 em. Preferably, the core of the introducer is of a diameter selected from the group consisting of: less than 3 mm; less than 2 mm; and less than 1.5 mm. Preferably, the introducer can bear an axial force of more than 20N; preferably more than SON. Preferably the introducer is harder than the implant and has a hardness selected from the group consisting of: 490 MPa (50 HV); 981 MPa (100 HV); and; 1961 MPa (200 HV).

Preferably, the distal end of the introducer is a sharp penetrative tip and wherein the tip can penetrate bone under compressive force or drilling. Preferably, the tip comprises between 1-5 faces and wherein each of the faces are of a slope angle of between 10-45 degrees from the centerline. Preferably the tip of the introducer is of a diameter selected from the group consisting of: the diameter of the tip is greater than 10% larger in diameter than the inner diameter of the implant; the diameter of the tip is greater than 30% larger in diameter than the inner diameter of the implant; the diameter of the tip is greater than 50% larger in diameter than the inner diameter of the implant; and the diameter of the tip is greater than 80% larger in diameter than the inner diameter of the implant.

Optionally, the tip comprises a plurality of tip parts and the tip parts each comprise an extraction rod extending along the core to the proximal end of the assembly. Optionally, the tip parts are extracted following extraction of the core by pulling the extraction rods. Optionally, the introducer is retracted through the same initial cavity. Optionally, the introducer blocks motion of the implant in the direction opposite to the insertion direction of the implant. Preferably, the introducer can bear between 15-125 LBS. of torque. Preferably, the introducer is stable at temperatures exceeding 80°C and preferably at temperatures exceeding 140°C.

Optionally, the implant is used in the treatment of one or more of: comminuted fractures, segmental fractures, non-union fractures, fractures with bone loss, proximal and distal fractures, diaphyseal fractures, or osteotomy sites. Optionally, the implant is used in a treatment selected from the list consisting of: load bearing bone fixation; non-load bearing bone fixation; bone fixation in parallel with an external support; and bone fixation with no external support.

Preferably, the biocomposite material is bioabsorbable. Preferably, the biocomposite material comprises a biocompatible absorbable polymer, reinforcement filler and a coupling agent. Optionally, the polymer is selected from the group consisting of: natural polymer, synthetic polymer, homo-polymer, co-polymer, terpolymer, polyesters, aliphatic polyesters, polyorthoesters, polyanhydrides, polycarbonates, polyurethanes, polyamides, polyalky lene oxides, polylactides (PLA), poly-L-lactide (PLLA), poly-DL-lactide (PDLLA); polyglycolide (PGA); copolymers of glycolide; glycolide/trimethylene carbonate copolymers; (PGA/TMC); and other polymers from the PLA family.

Optionally, the reinforcement filler is selected from the group consisting of: an organic material, an inorganic material; a mineral; a mineral chosen to increase the bioactivity of the implant; a mineral chosen to increase the mechanical strength of the implant; fibers; continuous fibers; a biodegradable glass, a cellulosic material, calcium phosphate, a nano-diamond, and a combination of any of the above. Preferably, the reinforcement filler comprises a volume of the implant selected from the group consisting of: more than 20% w/w; more than 40% w/w; and more than 45% w/w.

Preferably, the elastic modulus of the biocomposite material is selected from the group consisting of: 10 GPa, above 15 GPa, and above 20 GPa. Preferably, the elastic modulus of the biocomposite material is selected from the group consisting of: not exceeding 100 GPa; and not exceeding 60 GPa. Preferably, the flexural modulus of the implant is selected from the group consisting of: more than 200 MPa; more than 300 MPa; and more than 400 MPa. Preferably, the implant retains at least 50 % of its flexural strength and modulus for more than 6 weeks or preferably, the implant retains at least 50 % of its flexural strength and modulus for more than 12 weeks or preferably, the implant retains at least 50 % of its flexural strength and modulus for more than 24 weeks.

Preferably, the implant degrades in less than 24 months or preferably in less than 18 months or preferably in less than 12 months. Optionally, the assembly is introduced percutaneously. Optionally, the introducer is retracted through one of the distal end of the implant, the proximal end of the implant or both ends of the implant. Preferably, the assembly further comprises a release mechanism to allow separation of the introducer and the implant. Optionally, the release mechanism is selected from the list consisting of: cutting a distally welded or crimped area to release internal locking; leavers; a ratchet mechanism; and a combination of the above.

Optionally, extraction of the tip parts is performed by a mechanism selected from the group consisting of: linear motion; rotation motion; compression; compression release; tensioning; tension release; screws; springs; and any combination of the above. Optionally, the implant is formed as one of a: pin; wire, nail, screw, staple, plate; anchor; intramedullary nail; joint implant; spine implant, device for fracture fixation, device for tendon reattachment, device for spinal fixation, or a spinal cage. Optionally, the implant is formed as a screw and the screw is selected from the group consisting of: cannulated; threaded; not threaded; partially threaded; self-tapping; self-drilling; and a combination of the above. Optionally, the external screw diameter is between 0.5-3.0 mm. Optionally, the external screw diameter is between 5.0-8.0 mm. Optionally, the implant is formed as a pin of between 0.5-5.0 mm diameter and 2-25 cm in length. Optionally, the implant is formed as an anchor of between 1.0-6.5 mm diameter.

Optionally, the tip comprises foldable metal leaflets. Optionally, the tip comprises metal leaflets supported by a balloon. Optionally, the tip comprises retractable flaps extending over the implant. Optionally, the tip comprises an expanded construction element comprising metal tube segments cut in longitudinal strips. Optionally, the assembly further comprises a positioning device for positioning the assembly.

Preferably, the implant has a pull out strength from the cavity selected from the group consisting of: more than 2N; more than 20N; more than 60 N; and more than 100 N.

According to other embodiments of the present invention a method for introducing an orthopedic implant into a subject comprises providing the introducer assembly as described above; initiating a cavity by the introducer of the assembly; inserting the assembly into the cavity to thereby extend the cavity; retracting the introducer from the assembly to leave the implant in the cavity. Preferably, the method further comprises: where a portion of the implant extends out of the cavity, cutting the implant so that it does not extend from the cavity.

According to other embodiments of the present invention an introducer assembly for introducing an orthopedic implant into a subject comprises: an introducer comprising an elongated shaft with a sharpened tip on the distal end; and the implant, wherein the implant comprises a biocomposite material and the implant encloses the shaft for at least a portion thereof; wherein the introducer initiates a cavity for the implant, wherein the assembly is inserted into the cavity to thereby extend the cavity, and wherein the introducer is retracted from the assembly leaving the implant in the cavity.

According to other embodiments of the present invention a method for introducing an orthopedic implant into a subject comprises: providing an introducer assembly comprising an introducer and a biocomposite implant wherein the introducer comprises a shaft and a sharpened tip at the distal end of the shaft, wherein the implant encloses a portion of the shaft, wherein the outer diameter of the tip is greater than the inner diameter of the implant; initiating a cavity by the introducer of the assembly; inserting the assembly into the cavity to thereby extend the cavity; and retracting the introducer from the assembly to leave the implant in the cavity, wherein the retracting forces implant against the inner walls of the cavity.

According to other embodiments of the present invention a method for introducing an orthopedic implant into a subject comprises: providing an introducer assembly comprising an outer sleeve enclosing the majority of an inner shaft wherein the inner shaft comprises a sharpened tip at its distal end, wherein the inner shaft and the sleeve are joined at their proximal ends, wherein the sharpened tip protrudes from the sleeve; initiating a cavity in a bone of the subject by the tip of the assembly; inserting the assembly into the cavity to thereby extend the cavity; unjoining the shaft and the sleeve; retracting the sleeve to leave the shaft in the cavity; inserting a biocomposite implant onto the shaft followed by a holding sleeve; pushing the implant by the holding sleeve into the cavity guided by the shaft; and retracting the holding sleeve from the shaft and the shaft from within the implant to leave the implant in the cavity.

According to other embodiments of the present invention a method for introducing an orthopedic implant into a subject comprises: providing an introducer assembly comprising a threaded outer sleeve enclosing the majority of an inner shaft wherein the inner shaft comprises a sharpened tip at its distal end, wherein the inner shaft and the sleeve are joined at their proximal ends, wherein the sharpened tip protrudes from the sleeve; initiating a cavity in a bone of the subject by the tip of the assembly; screwing the assembly using the threaded sleeve into the cavity to thereby extend the cavity and form a threaded cavity; unjoining the shaft and the sleeve; retracting the sleeve to leave the shaft in the cavity; inserting a threaded biocomposite implant onto the shaft followed by a holding sleeve; wherein the implant matches the dimensions of the threaded sleeve; screwing the implant into the threaded cavity guided by the shaft; and retracting the holding sleeve from the shaft and the shaft from within the implant to leave the implant in the cavity.

The term "biodegradable" as used herein refers to materials that are resorbable or absorbable in the body.

The term "load bearing" optionally also includes partially load bearing. According to various embodiments, the load bearing nature of the device may optionally be above 200 MPa, preferably above 300 MPa, more preferably above 400.

The term distal as used herein refers to the front end or tip of the assembly that initially penetrates the bone. The term proximal as used herein refers to the back end of the assembly that is generally manipulated by medical personnel.

Implementation of the method and system of the present invention involves performing or completing certain selected tasks or steps manually, automatically, or a combination thereof.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIGS. 1A-1D are cross sectional schematic illustrations of an introducer assembly used to repair a bone fracture according to at least some embodiments of the present invention;
FIGS. 2A-2C are schematic diagrams of introducer assemblies adapted for different implant types according to at least some embodiments of the present invention;
FIG. 3 is a schematic diagram of an introducer assembly according to some embodiments of the present invention;
FIGS. 4A-4D are schematic diagrams of an introducer assembly according to some embodiments of the present invention;
FIGS. 5A-5D are schematic diagrams of an introducer assembly according to some embodiments of the present invention;
FIGS. 6A-6C are schematic diagrams of an introducer assembly according to some embodiments of the present invention;
FIGS. 7A-7B are schematic diagrams of an introducer assembly according to some embodiments of the present invention;
FIGS. 8A-8B are schematic diagrams of an introducer assembly according to some embodiments of the present invention;
FIGS. 9A-9D are schematic diagrams of an introducer assembly according to some embodiments of the present invention;
FIGS. 10A-10D are schematic diagrams of an introducer assembly according to some embodiments of the present invention;
FIG. 11 is a schematic diagram of an introducer assembly according to some embodiments of the present invention;
FIGS. 12A-12B are schematic diagrams of an introducer assembly according to some embodiments of the present invention;
FIG. 13 is a schematic diagram of an introducer assembly according to some embodiments of the present invention;
FIGS. 14A-14C are schematic diagrams of an introducer assembly according to some embodiments of the present invention;
FIGS. 15A-15D are schematic diagrams of an introducer assembly according to some embodiments of the present invention;
FIGS. 16A-16C are schematic diagrams of an introducer assembly with an implant cut to size once positioned according to some embodiments of the present invention; and
FIG. 17 is a schematic diagram of an introducer assembly positioning device according to some embodiments of the present invention.

### DESCRIPTION OF THE PREFERRED EMBODIMENTS

The present invention is of an introducer assembly for inserting reinforced biocomposite biodegradable implants into bone and soft tissue of human or non-human subjects. The introducer assembly comprises an introducer and a biocomposite implant enabling implant insertion as part of a single step or in fewer steps than required in prior art methods. The introducer assembly enables insertion of orthopedic implants made from reinforced bioabsorbable biocomposite materials for structural fixation, such as for bone fixation including in load-bearing or non-load-bearing situations and/or in parallel or not in parallel with external support such as casts or similar supports.

The introducer assembly incorporates characteristics, features, or properties that can either only be achieved using the biocomposite materials as part of the assembly or are specifically advantageous for implants comprising these types of materials. Without wishing to be limited by a closed list, the introducer assembly provides the following functionality and benefits:
1. The assembly can be introduced in one step into bone, without prior preparation;
2. The structural properties of the biocomposite implant contribute to the penetration into the soft and bone tissue;
3. In some embodiments the biocomposite implant widens the hole created by the sharp tip of the introducer.
4. The introducer can be repositioned when needed and disconnected from either the proximal or distal side of the implant after positioning leaving the implant fixed in the desired position;
5. The introducer that penetrates the bone is of an external diameter less than external diameter of the implant ensuring a tight fit for the implant.

Embodiments of the invention described herein may be manufactured in any desired physical form to support the required application. The introducer may be manufactured for example, by welding, laser cutting, machining, or any other manufacturing technique known in the art. Furthermore, it is preferable to produce the implant with sufficient robustness to provide the necessary mechanical strength but otherwise not contain extraneous material.

The introducer and the implants preferably have a minimal profile so as to allow for implantation with minimal damage to both soft and bone tissue. Each embodiment and/or application has its own preferable properties. In all cases the introducer sizes are based on the implant size where the implant will preferably have the minimum profile that will be inherently strong enough to maintain the fixation.

Preferably, the introducer is of external diameter less than external diameter of the implant. In some embodiments the shaft of the introducer may pass through the center of the hollow implant. Such a structure ensures that the implant has maximal contact with the surrounding bone.

Preferably, the introducer is at least partially comprised of a material that is harder than the implant. Preferably, the harder material is at least two times as hard as the implant material, more preferably at least three times as hard. Preferably, the harder material is at least of 50 Vickers hardness (HV), or preferably at least 100 HV, or preferably at least 200 HV.

Preferably, the introducer includes a sharp penetrative point or trocar capable of penetrating bone under compressive force or alternatively by drilling.

Preferably the introducer can be retracted from the same location through which it was inserted, minimizing tissue damage and decreasing the chances of inflammation due to skin penetration.

Preferably, the introducer blocks motion of the implant in the direction opposite to the insertion direction of the implant. Preferably the insertion device may be used percutaneously.

Preferably, the introducer shaft diameter is less than 80% of the outer diameter of the implant, or preferably it is less than 60%.

The material of the introducer is preferably one of stainless steel 316 L, 316 LVM, 304, Titanium, Nitinol or any other biocompatible metal.

The length of the introducer is preferably 2-30 em, more preferably 5-20 em and most preferably 8-15 em.

The diameter of the shaft of the introducer is preferably less than 3 mm, more preferably less than 2 mm and preferably less than 1.5 mm.

The introducer can preferably bear a load of more than 20N when inserted, more preferably more than SON.

Preferably the tip of the inserter is sharp and made of 1-5 faces. Preferably the faces are of a slope angle of less than 45 degrees from the centerline, more preferably less than 30 degrees, and most preferably less than 16 degrees.

Preferably the introducer can withstand at least 1.7 N^{∗}M (15 in-lbs.) of torque, more preferably 3.4 N^{∗}m (30 in-lbs.) and most preferably 14.1 N^{∗}m (125 in-lbs.)

Preferably the material of the introducer is stable at elevated temperatures. Preferably the material is mechanically stable at elevated temperatures. Preferably at more than 80°C, preferably at more than 100°C and most preferably at more than 140°C.

The implant is made from an absorbable material. Preferably a reinforced composite, consisting of a combination of biocompatible absorbable polymer, reinforcement filler and coupling agent.

The biodegradable polymer portion is preferably of natural or synthetic origin, homo-polymer or co-polymer or terpolymer and preferably consists of aliphatic polyesters, polyorthoesters, polyanhydrides, polycarbonates, polyurethanes, polyamides and polyalky lene oxides. Copolymer or terpolymer preferably include: polylactides (PLA), poly-L-lactide (PLLA), poly-DL-lactide (PDLLA); polyglycolide (PGA); copolymers of glycolide, glycolide/trimethylene carbonate copolymers (PGA/TMC) or other polymers from the PLA family.

The reinforcement filler preferably may comprises organic or inorganic (that is, natural or synthetic) material. Preferably the reinforcement filler is a mineral. Preferably the mineral based reinforcement increases the bioactivity if the implant. Preferably the mineral reinforcement increases the mechanical strength of the implant. Preferably the minerals are in the form of fiber, preferably in the form of continuous fibers. Preferably the reinforcing filler is a biodegradable glass, a cellulosic material, a nano-diamond, or any other filler known in the art to increase the mechanical properties of a bioabsorbable polymer.

Preferably the filler portion is more than 20% w/w, preferably more than 40% w/w, and most preferably more than 45% w/w.

Preferably the implant is load bearing. Preferably the Elastic Modulus (or Young's Modulus) of the bioabsorbable composite for use with present invention is preferably at least 10 GPa, more preferably above 15 GPa, and even more preferably above 20 GPa but not exceeding 100 GPa and preferably not exceeding 60 GPa.

In some embodiment of the invention the implant is designed to resist bending. Preferably the flexural modulus of the implant is more than 200 MPa, preferably more than 300 MPa and most preferably more than 400 MPa.

Preferably the implant maintains its mechanical properties for an extended period to allow for sufficient bone healing, preferably it retains at least 50 % of its flexural strength and modulus for more than 6 weeks, preferably for more than 12 weeks and most preferably for more than 24 weeks.

Preferably the implant completely degrades when no longer required, preferably in less than 24 months, preferably in less than 18 months and most preferably in less than 12 months.

The Implant size preferably depends on the application and preferably has the minimum profile required to meet the needed mechanical properties for the specific application for the desired length of time.

In one embodiment of the invention, the medical implant is a pin. The pin is preferably a hollow pin, and preferably has a length of 2-10 cm, and more preferably e a length of 2-5 cm. Preferably the wall thickness of the pin is less than 3 mm, preferably less than 1.5 mm and preferably less than 0.8 mm.

In one embodiment of the invention, the medical implant preferably has an external diameter of less than 6mm, preferably less than 4 mm and most preferably less than 2.5mm.

In one embodiment of the herein invention, the medical implant is a screw. Preferably a cannulated screw, preferably with a length of 2-10 cm, more preferably with a length of 2-5 cm. Preferably the screw will have an outer diameter of less than 6mm, or preferably less than 4 mm. Preferably the inner diameter of the cannulated hole in the screw is more than 0.6 mm, or preferably more than 0.8 mm and in some cases preferably more than 2 mm.

According to at least some embodiments, when the implant is inserted assembled onto the introducer the implant is later released either from the distal side or the proximal side or a combination thereof. Several optional release mechanisms exist. Without wishing to be limited by a closed list, these include cutting a distally welded or crimped area to release internal locking, leavers, ratchet mechanisms, etc. Once the internal locking is released, (and when needed, the distal introducer tip is collapsed) the parts of the assembly may be separated. Since the implant preferably has an increased pullout force, the introducer parts are removed while implant remains in place.

Preferably the introducer is removed through the insertion hole. In some embodiments the distal head must be collapsed or disassembled prior to complete retraction from the proximal side. Several mechanisms exist for such manipulations, including but not limited to techniques based on linear motion, rotation motion, compression or release thereof, tension or release thereof, screws, springs and any combination thereof. A non-limiting example for a release of compression forces can be the cutting of parts welded together to create the compression forces in the distal end.

The introducer assembly described herein is used for bone fracture reduction and fixation to restore anatomical relationships in human or non-human subjects. Such fixation optionally and preferably includes one or more, and more preferably all, of stable fixation, preservation of blood supply to the bone and surrounding soft tissue, and early, active mobilization of the part and patient.

The introducer assemblies described herein may optionally be used to fixate various fracture types including but not limited to comminuted fractures, segmental fractures, non-union fractures, fractures with bone loss, proximal and distal fractures, diaphyseal fractures, osteotomy sites, or similar.

In use the implant is introduced into the bone as part of the introducer assembly. The introducer penetrates the bone together with the implant but is then removed such that only the implant remains behind in the bone.

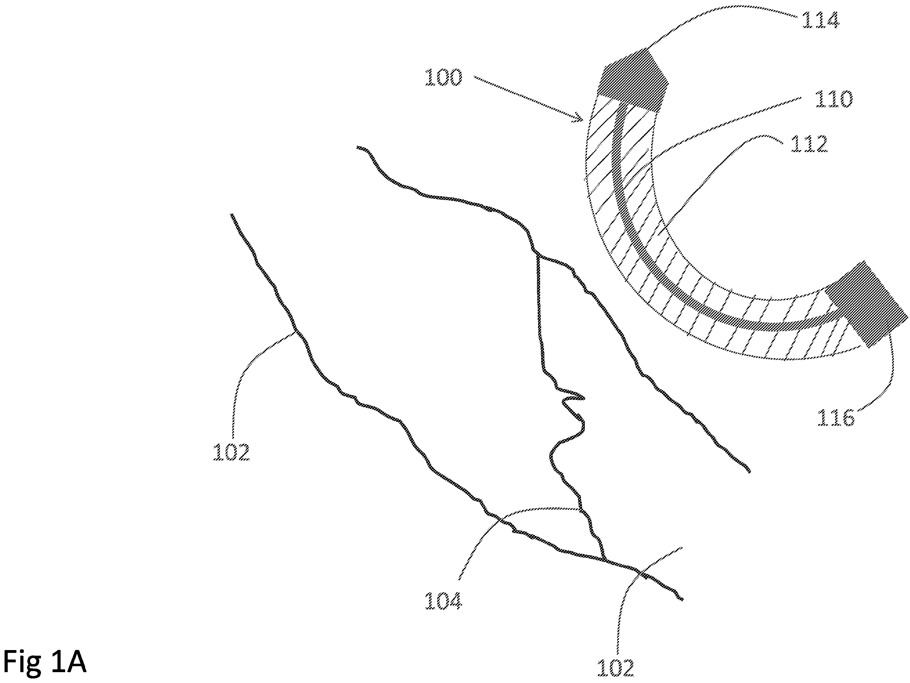

Reference is now made to figures 1A-1D which are cross sectional schematic illustrations of an introducer assembly used to repair a bone fracture according to at least some embodiments of the present invention. As shown in figure 1A, assembly 100 is prepared for insertion within bone 102 which is fractured as shown by fracture line 104. Assembly 100 comprises introducer 110 and implant 112. Distal end 114 of introducer 110 is sharpened at introducer tip 114 to enable the making of a hole in the bone for insertion of the assembly. Proximal end 116 is manipulated to insert assembly 100.

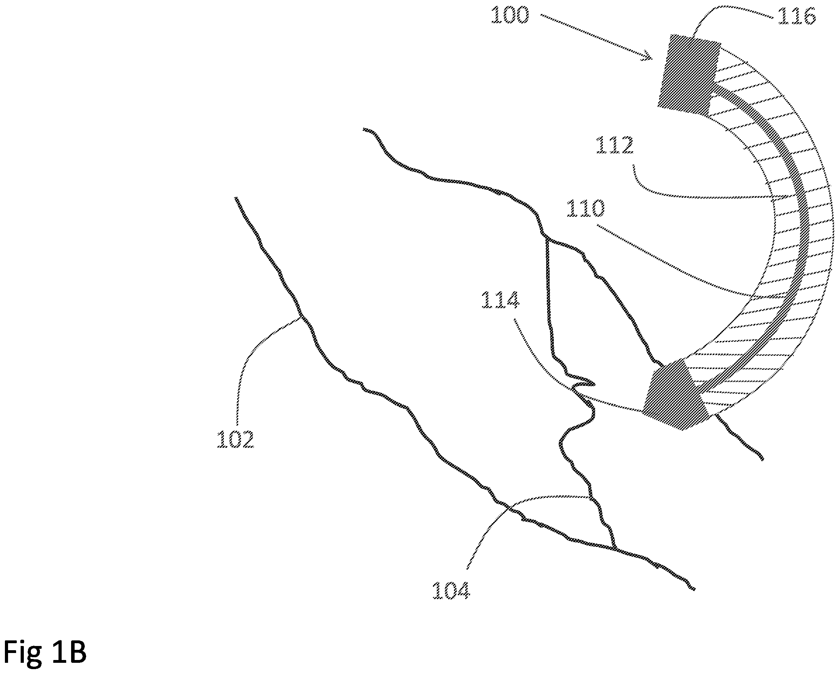

As shown in figure 1B, assembly 100 is inserted by making a hole in the bone 102 using sharpened introducer tip 114. The making of the hole is done by one of hammering on the proximal end 116 of introducer 110 to force tip 114 into the bone, or rotating end 116 mechanically or manually so that tip 114 rotates and drills into the bone.

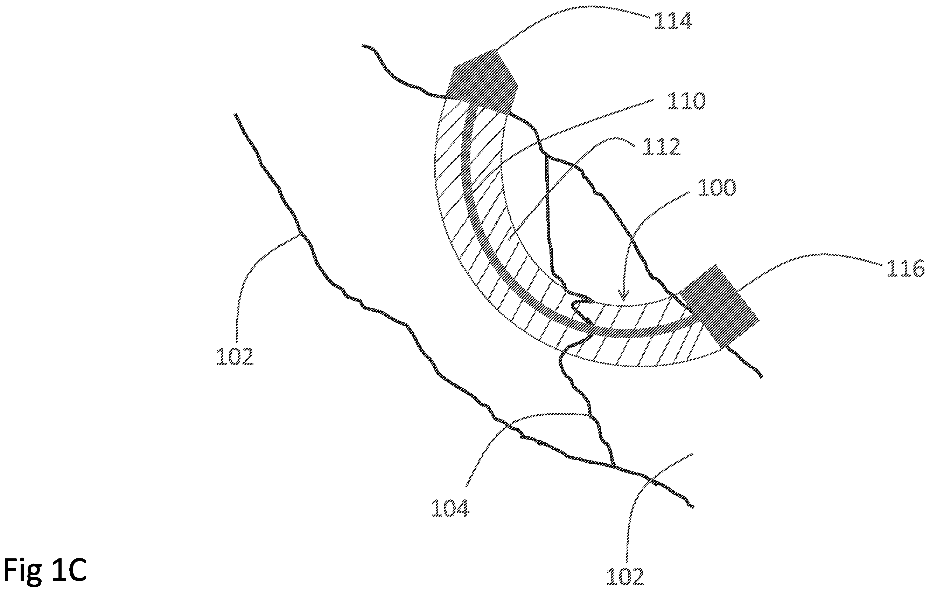

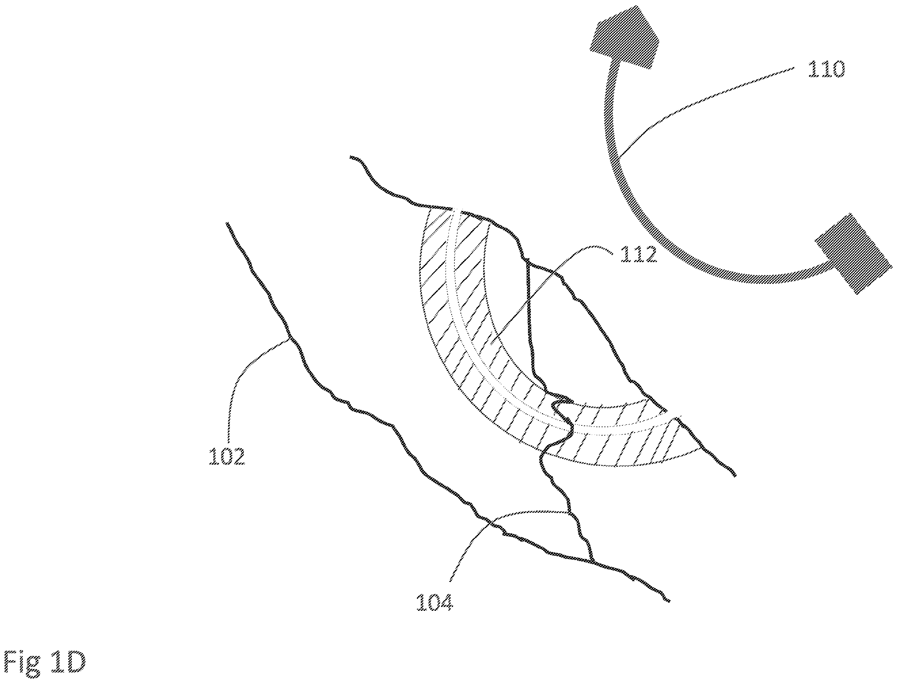

Once in place, as shown in figure 1C, assembly 100 joins the fractured bone 102 together. Introducer 110 is now removed and as shown in figure 1D implant 112 is left in place. Implant 112 then degrades over time as fracture 104 heals. The method of removal of introducer 110 is further described below and is dependent on the embodiment.

Embodiments of the invention described herein may be used for insertion of implants formed as pins, nails, screws, staples, and plates for bone fixation; intramedullary nails, joint (hip, knee, and elbow) implants, spine implants, and other devices for such applications such as for fracture fixation, tendon reattachment, spinal fixation, and spinal cages. Some of these implant types are further described below. It should be appreciated that different features of different embodiments presented herein may be combined in different ways. In particular, not all the features shown in a particular embodiment are necessary in every embodiment of the invention. Further combinations of the features of any embodiment with features of other embodiments are also considered to be within the scope of the invention.

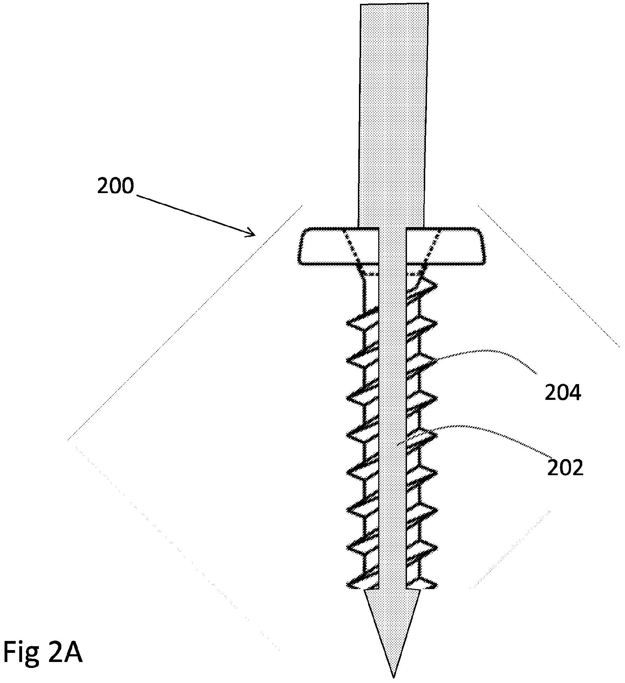

Reference is now made to figures 2A-2C which are schematic diagrams of introducer assemblies adapted for different implant types according to at least some embodiments of the present invention. Figure 2A shows an introducer assembly 200 comprising an introducer 202 and an implant 204 formed as a screw. Screws are used for internal bone fixation and there are different designs based on the type of fracture and how the screw will be used. Screws come in different sizes for use with bones of different sizes. Screws can be used alone to hold a fracture, as well as with plates, rods, or nails.

Screws are threaded, though threading can optionally be either complete or partial. Screws can optionally include compression screws, locking screws, and/or cannulated screws. The external screw diameter is preferably as small as 0.5 or 1.0 mm but is optionally less than 3.0 mm for smaller bone fixation. Larger bone cortical screws are preferably of a diameter of up to 5.0mm and cancellous screws are preferably 7-8 mm. Optionally, screws are self-tapping. Optionally, screws require drilling prior to insertion of the screw such as provided by introducer 202. Optionally the introducer is threaded or optionally it is not threaded (as shown). For cannulated screws, a hollow section in the middle is preferably larger than 0.8 mm diameter in order to accommodate guide wires.

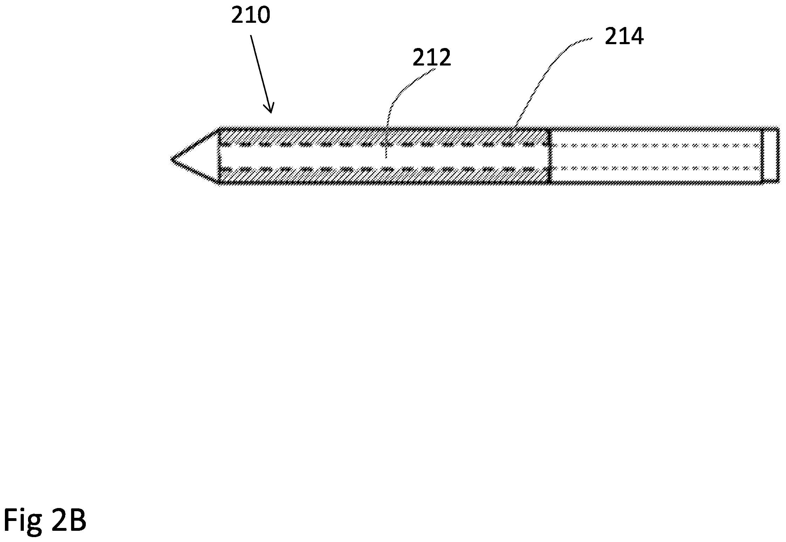

Figure 2B shows an introducer assembly 210 comprising an introducer 212 and an implant 214 formed as a wire or pin. The terms pin and wire for bone fixation are used herein interchangeably. Wires are often used to pin bones back together. They are often used to hold together pieces of bone that are too small to be fixed with screws. They can be used in conjunction with other forms of internal fixation, but they can be used alone to treat fractures of small bones, such as those found in the hand or foot. Typical dimensions of pins or wires are in the range of 0.5 - 2.0 mm diameter and 2-25 cm length. Pins are preferably in the range of 2.0 - 5.0 mm diameter and 2-25 cm length.

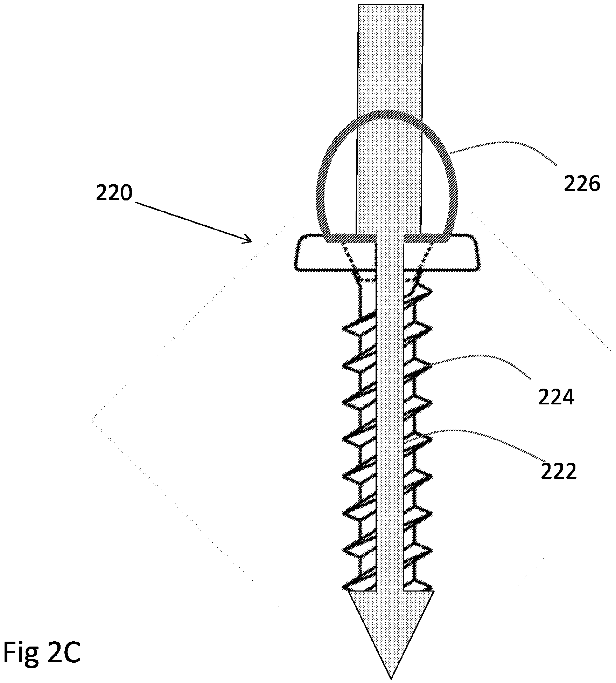

Figure 2C shows an introducer assembly 220 comprising an introducer 222 and an implant 224 formed as an anchor with a loop 226. Anchors and particularly suture anchors are fixation devices for fixing tendons and ligaments to bone. They are comprised of an anchor mechanism such as implant 224, which is inserted into the bone, and one or more eyelets, holes or loops such as loop 226 in the anchor through which the suture (not shown) passes. This links the anchor to the suture. The anchor which is inserted into the bone may optionally be a screw mechanism as shown or an interference mechanism. Anchors are preferably in the range of 1.0 - 6.5 mm diameter

Without wishing to limit the invention to specific implant types, some of the following introducer assembly embodiments are for a wire or pin, but it should be appreciated that the same structural principles can be applied to other implants types such as the screw, anchor or any other implant type.

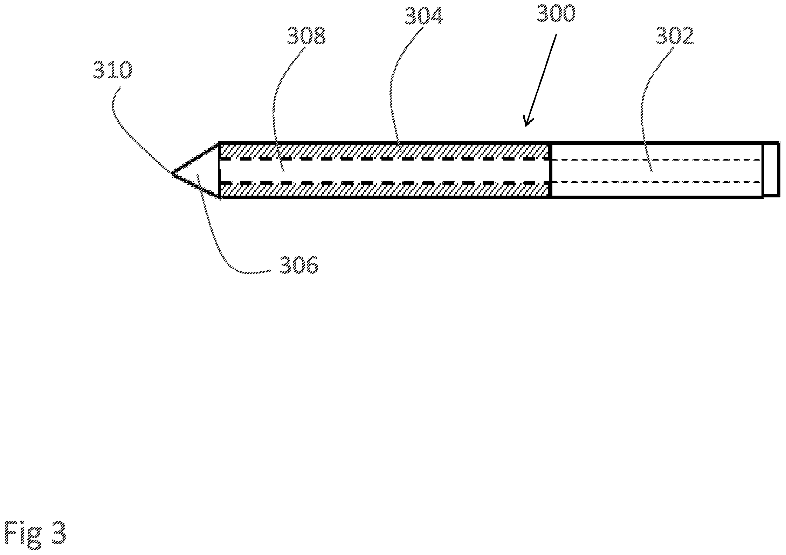

Reference is now made to figure 3 which is a schematic diagram of an introducer assembly according to some embodiments of the present invention. As shown in figure 3, the introducer 302 of an introducer assembly 300 has more than one diameter. The diameter of shaft 308 of introducer 302 is less than the outer diameter of implant 304 by at least 30%, preferably by 50%, and more preferably by 75%. The second maximum diameter, such as for the introducer tip 306, is preferably the same or greater than the outer diameter of the implant 304 or alternatively not more than 30% less than the outer diameter of the implant 304 and preferably not more than 15% less.

Preferably, the introducer 302 includes a sharp point 310 of tip 306 that can be used as an insertion point for either being hammered, screwed or drilled into bone. Preferably, sharp point 310 comprises at least one face but also preferably fewer than five faces (not shown). Preferably the faces are of slope angle less than 30 degrees from centerline, more preferably less than 20 degrees, and most preferably less than 16 degrees.

Preferably, the introducer 302 incorporating sharp point 310 can be separated from implant 304 and retracted following insertion of the implant 304 into the bone.

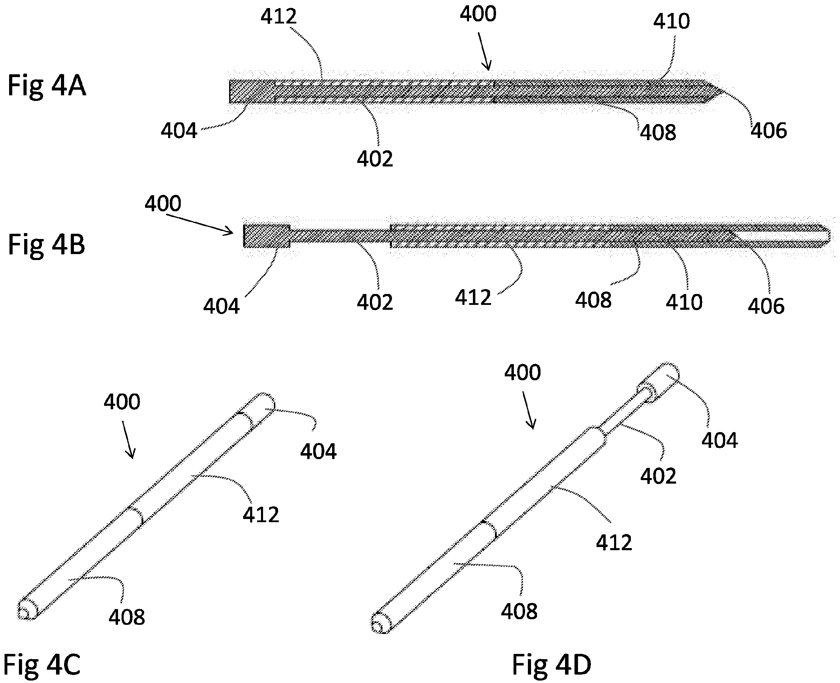

Reference is now made to figures 4A-4D which are schematic diagrams of an introducer assembly according to some embodiments of the present invention. Figures 4A-4D show an introducer assembly 400 with an introducer 402 with a handle 404 to prevent slippage during insertion. The introducer 402 comprises a sharp tip 406 to pierce the bone surface. The absorbable implant 408 sits on the introducer shaft 410. In use the introducer 402 pierces the bone surface and is pushed into the bone as shown above with reference to figures 1A-1D. Once implant 408 is fully in the bone, the surgeon holds onto the introducer sleeve 412 to keep the implant 408 in place and then removes the introducer 402 by holding onto the handle 404 and pulling it out of the bone. Figures 4A and 4C show the introducer assembly 400 with the introducer 402 fully inserted into implant 408. Figures 4B and 4D show the introducer assembly 400 with the introducer 402 partially extracted from implant 408.

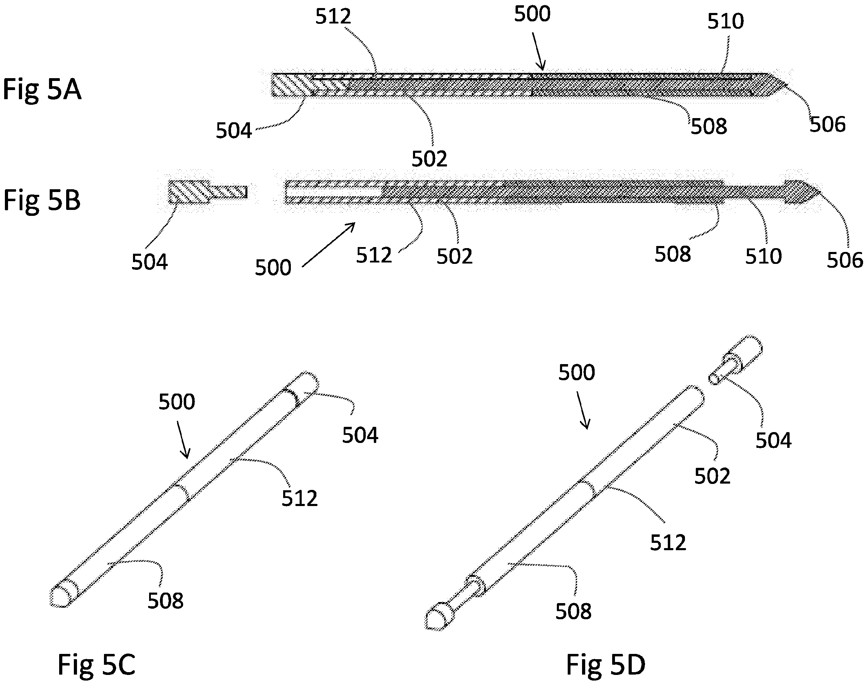

Reference is now made to figures 5A-5D which are schematic diagrams of an introducer assembly according to some embodiments of the present invention. Figures 5A-5D show an introducer assembly 500 with a detachable introducer 502. The absorbable pin implant 508 sits on the introducer 502 and a sharp tip 506 that is larger than the inner diameter of implant 508 extends from the introducer 502 out to the distal side of the implant 508. In use the sharp tip 506 of the introducer 502 pierces the bone surface and is pushed into the bone. Once implant 508 is in position in the bone, the surgeon holds onto the introducer sleeve 512 and then proceeds to remove the introducer handle 504 from the proximal side of the assembly 500 and the remainder of the shaft of the introducer 502 including the sharp tip 506 from the distal side of the assembly 500. Sharp tip 506 is preferably >10%, more preferably, > 30%, or more preferably >50% larger in diameter than the inner diameter of implant 508 and therefore has to be pushed out the distal side of assembly 500 and removed from there.

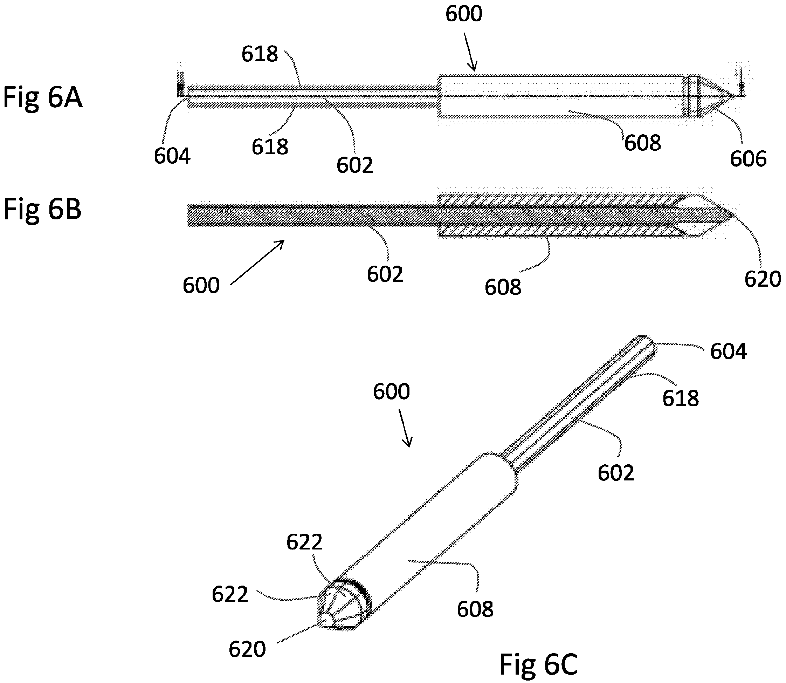

Reference is now made to figures 6A-6C which are schematic diagrams of an introducer assembly according to some embodiments of the present invention. Figures 6A-6C show an introducer assembly 600 with an introducer 602 with an assembled cone shape tip 606, featuring a sharp tip core 620 and 8 smaller tip parts 622 which together form tip 606. Core 620 forms the central core of introducer 602 extending back to the proximal end 604 of introducer 602. Tip parts 622 each comprise extraction rod 618 which extends back along core 620 to proximal end 604. Once core 620 is retracted from implant 608, all other tip parts 622 can be removed by grasping and pulling each of extraction rods 618 through the cavity vacated by core 620 which is big enough for each of the parts 622 to pass through. Thus all of introducer 602 is removed.

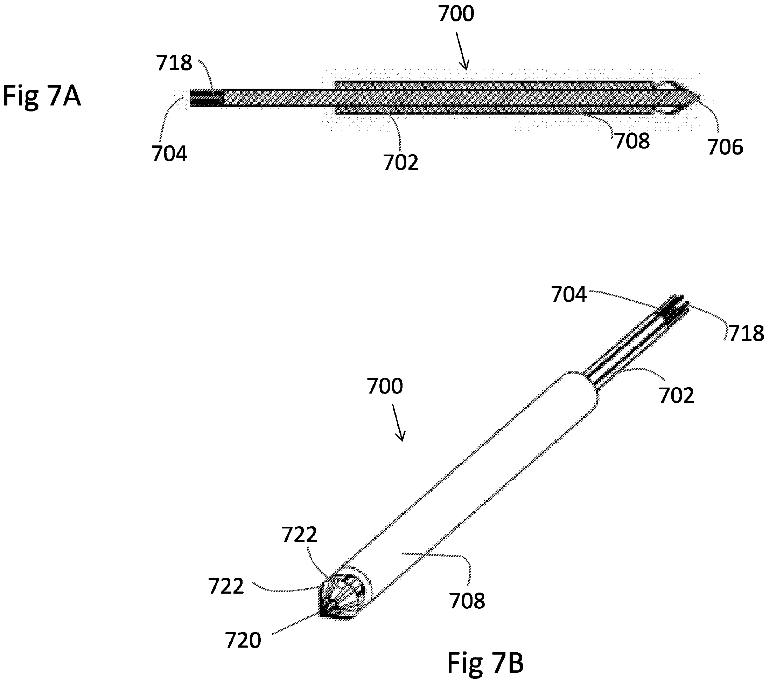

Reference is now made to figures 7A-7B which are schematic diagrams of an introducer assembly according to some embodiments of the present invention. Figures 7A-7B show an introducer assembly 700 with an introducer 702 with an assembled pyramid like shape tip 706, featuring a sharp tip core 720 and 6 smaller tip parts 722, each with a fin base and three faces which together form tip 706. Core 720 forms the central core of introducer 702 extending back to the proximal end 704 of introducer 702. Tip parts 722 each comprise extraction rod 718 which extends back along core 720 to proximal end 704. Once core 720 is retracted from implant 708, all other tip parts 722 can be removed by grasping and pulling each of extraction rods 718 through the cavity vacated by core 720 which is big enough for each of the parts 722 to pass through. Thus all of introducer 702 is removed.

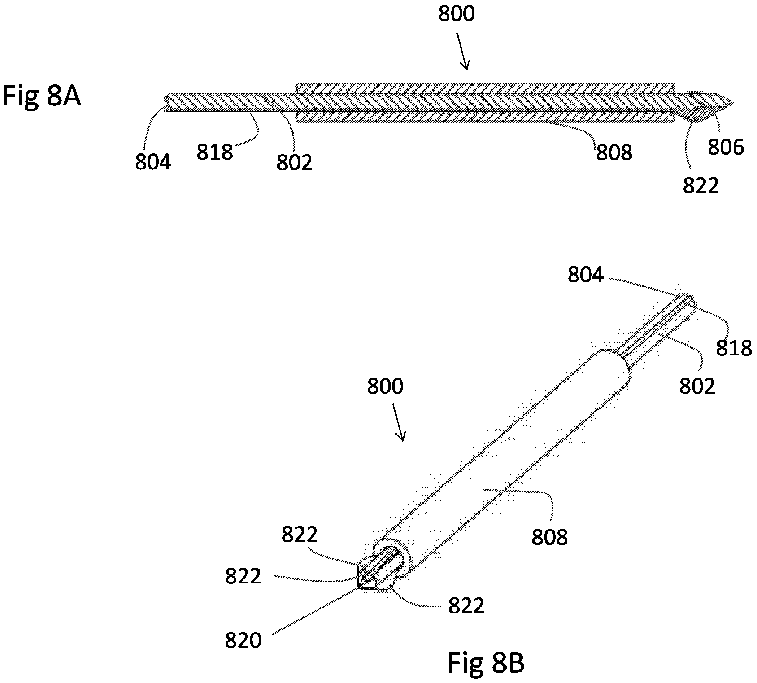

Reference is now made to figures 8A-8B which are schematic diagrams of an introducer assembly according to some embodiments of the present invention. Figures 8A-8B show an introducer assembly 800 with an introducer 802 with an assembled tip 806 composed of a sharp core 820 and 3 sharp blades 822. Core 820 forms the central core of introducer 802 extending back to the proximal end 804 of introducer 802. Tip parts 822 each comprise extraction rod 818 which extends back along core 820 to proximal end 804. Once core 820 is retracted from implant 808, all other tip parts 822 can be removed by grasping and pulling each of extraction rods 818 through the cavity vacated by core 820 which is big enough for each of the parts 822 to pass through. Thus all of introducer 802 is removed.

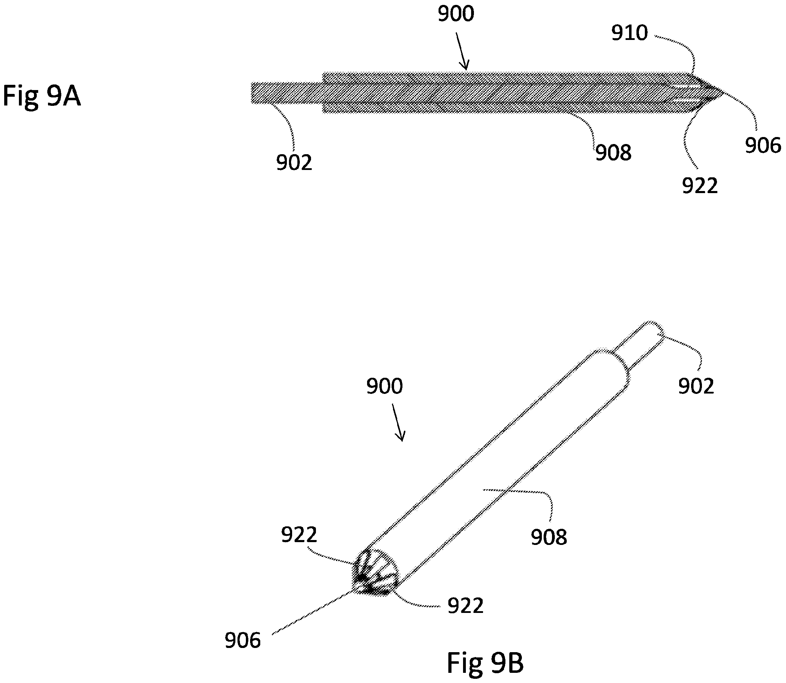

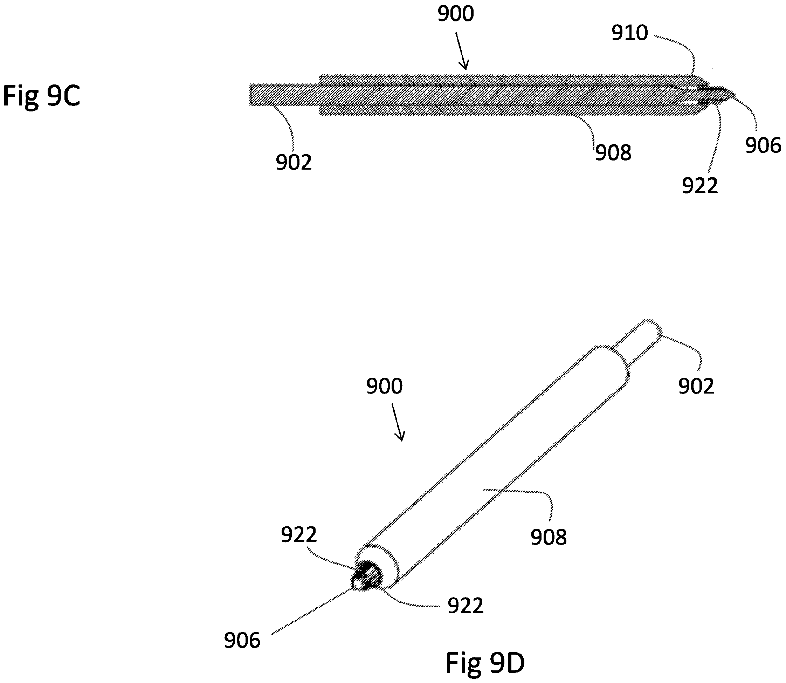

Reference is now made to figures 9A-9D which are schematic diagrams of an introducer assembly according to some embodiments of the present invention. Figures 9A-9B show an introducer assembly 900 with an introducer 902 comprising an umbrella penetrating sharp tip 906. The additional metal leaflets 922 cover the hollow implant 908 to fully or partially shield the implant 908 during the insertion process. In figures 9C-9D assembly 900 is shown with leaflets 922 folded onto the main stem 910 of introducer 902 (in a similar manner to an umbrella) ready for retraction. Once folded, the introducer 902 can be retracted through the center cavity of implant 908. To enable folding, either the implant 908 needs to be slightly pulled back or the introducer 902 needs to be pushed forward slightly to expose stem 910 and enable folding. The introducer pushed against the bone holds the umbrella open during insertion. The implant is held in place on the shaft 902 from behind by an additional sleeve (part not shown) similar to those embodiments shown with reference to figures 4, 5, and 14.

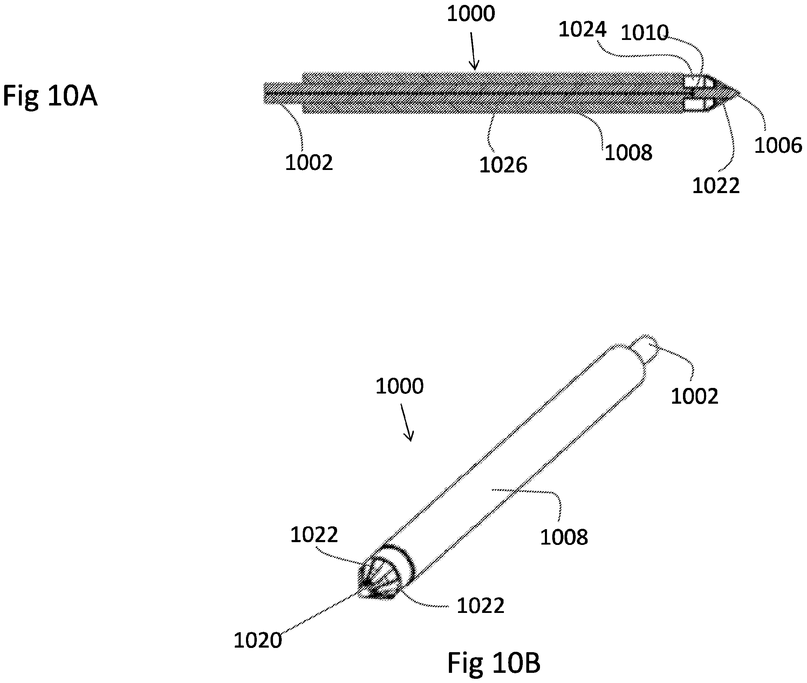

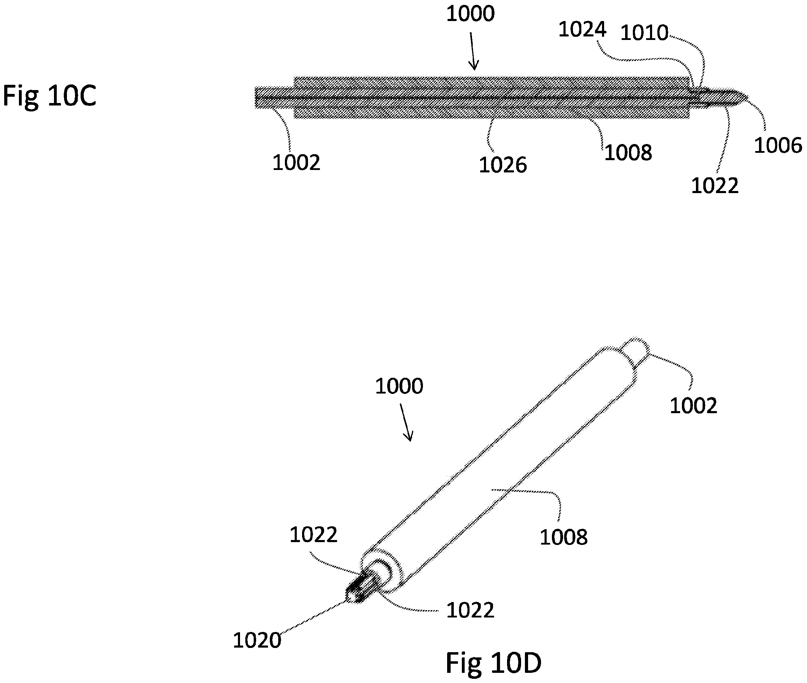

Reference is now made to figures 10A-10D which are schematic diagrams of an introducer assembly according to some embodiments of the present invention. Figures 10A-10B show an introducer assembly 1000 with an introducer 1500 comprising an umbrella penetrating sharp tip 1006 with a balloon mechanism, featuring a sharp center metal tip 1020 with additional metal leaflets 1022 which are supported by an inflated balloon 1024. In figures 10C-10D assembly 1000 is shown with leaflets 1022 folded onto the main stem 1010 of introducer 1002 once balloon 1024 is deflated (in a similar manner to an umbrella) ready for retraction. Once folded, the introducer 1002 can be retracted through the center cavity of implant 1008. Optionally, compressed gas is introduced into the balloon through a narrow tube 1026 inside the assembly 1002.

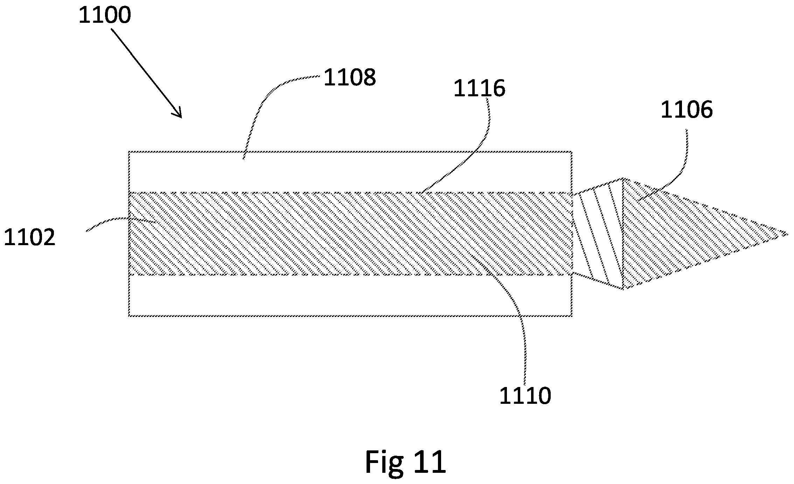

Reference is now made to figure 11 which is a schematic diagram of an introducer assembly according to some embodiments of the present invention. Figure 11 shows an introducer assembly 1100 with an introducer 1102 comprising sharp tip 1106 wherein the outer diameter of tip 1106 is greater than the inner diameter of the hollow implant 1108 but less than the outer diameter of the implant 1108. Preferably diameter of tip 1106 is <50% larger than the diameter of introducer shaft 1110. More preferably tip 1106 is < 30% larger and most preferably tip 1106 is < 20% larger. When tip 1106 is retracted through implant 1108, the larger diameter of tip 1106 pushes on the inner wall 1116 of the implant 1108 forcing implant 1108 towards the surrounding bone surface (not shown), increasing friction with the bone and thus improving anchoring of implant 1108 in the bone. Tip 1106 may optionally not be threaded at all, threaded only on penetrating side, or threaded only on retracting side (as shown).

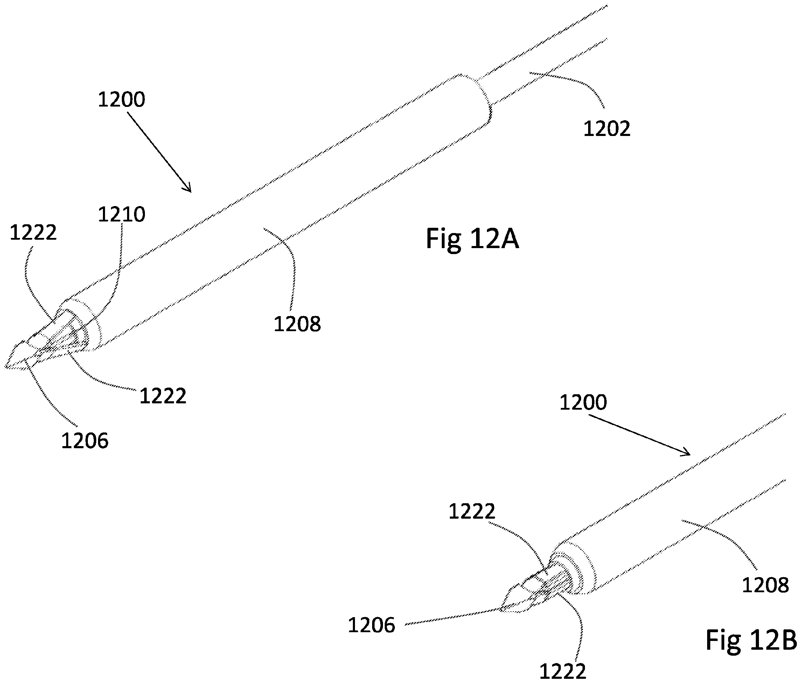

Reference is now made to figures 12A-12B which are schematic diagrams of an introducer assembly according to some embodiments of the present invention. Figures 12A-12B show an introducer assembly 1200 with implant 1208 and introducer 1202. Implant 1208 sits on the shaft 1210 of introducer 1202. As shown in figure 12A, at the distal end of introducer 1202 is a sharp tip 1206 additionally containing three flaps or wings 1222 such that the flaps 1222 extend over the implant 1208 when the implant 1208 is assembled onto introducer 1202 and pushed up under the flaps 1222 such that they partially cover the front end of implant 1208. As shown in figure 12B when the introducer 1202 is pushed out from the implant 1208, the flaps 1222 return to their preconfigured position and close down such that tip 1206 together with flaps 1222 can be withdrawn through the hollow center of the implant 1208.

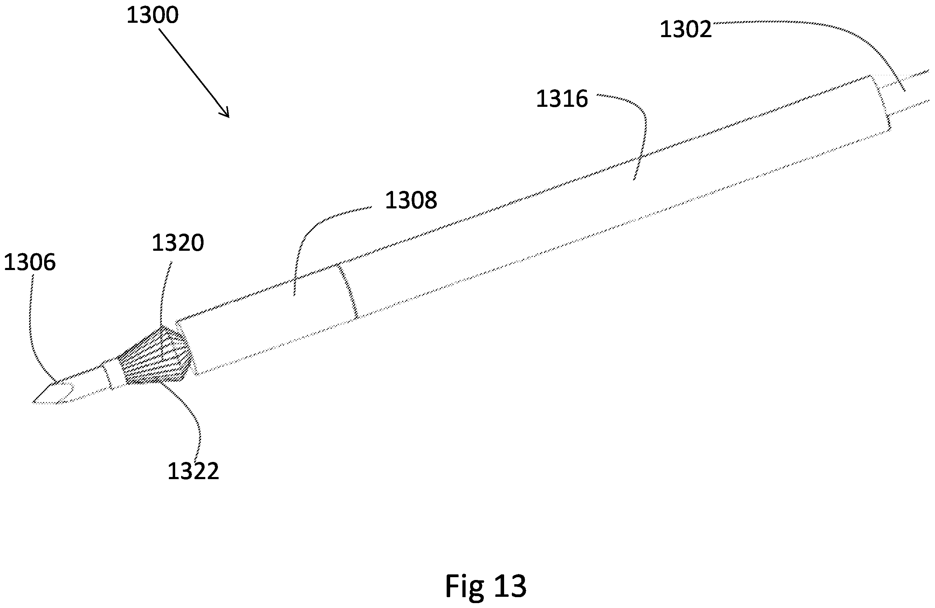

Reference is now made to figure 13 which is a schematic diagram of an introducer assembly according to some embodiments of the present invention. Figure 13 shows an introducer assembly 1300 comprising implant 1308 and introducer 1302. Implant 1308 sits on the shaft of introducer 1302. At the distal end of introducer 1302 is a sharp tip 1306 comprising an expanded construction element 1320 which is comprised of metal tube segments 1322 cut in longitudinal strips such that when element 1320 is expanded, its diameter is greater than the inner diameter of implant 1308. When the element 1320 is collapsed, for example by application of mechanical force, (which may be based on unscrewing a mechanism, or pushing the shaft forward relative to the implant 1308 so that element 1320 has room to collapse) the element 1320 collapses and is able to fit through the inner diameter of the implant 1308 such that the introducer 1302 may be withdrawn. This assembly additionally comprises an introducer sleeve handle 1316 that sits behind the implant 1308 such that the handle 1316 can be grasped and held to make it easier to withdraw the tip 1306 of the introducer 1302 through the inner diameter of implant 1308.

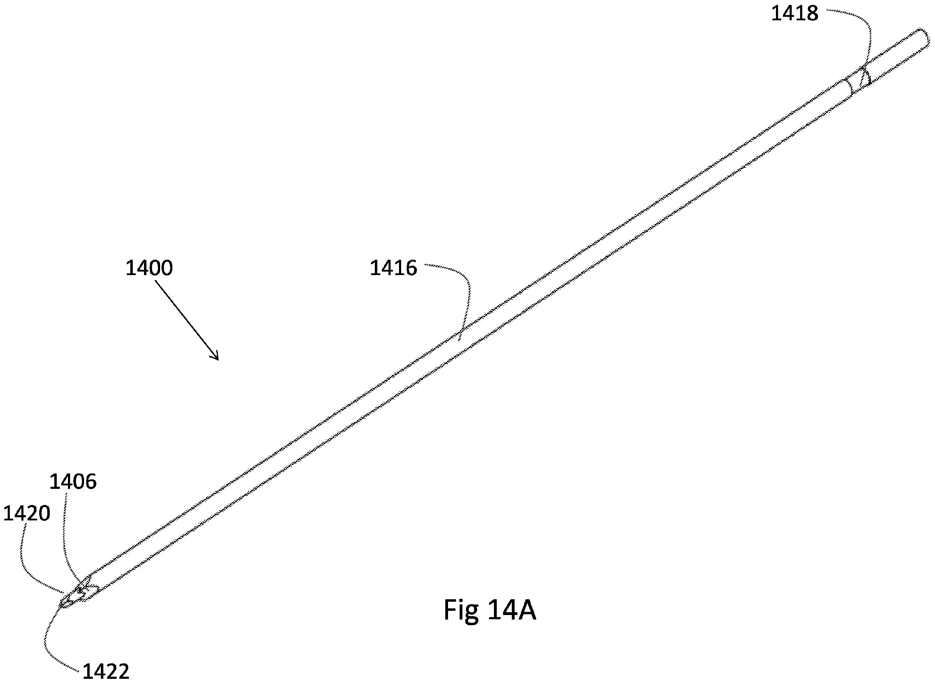

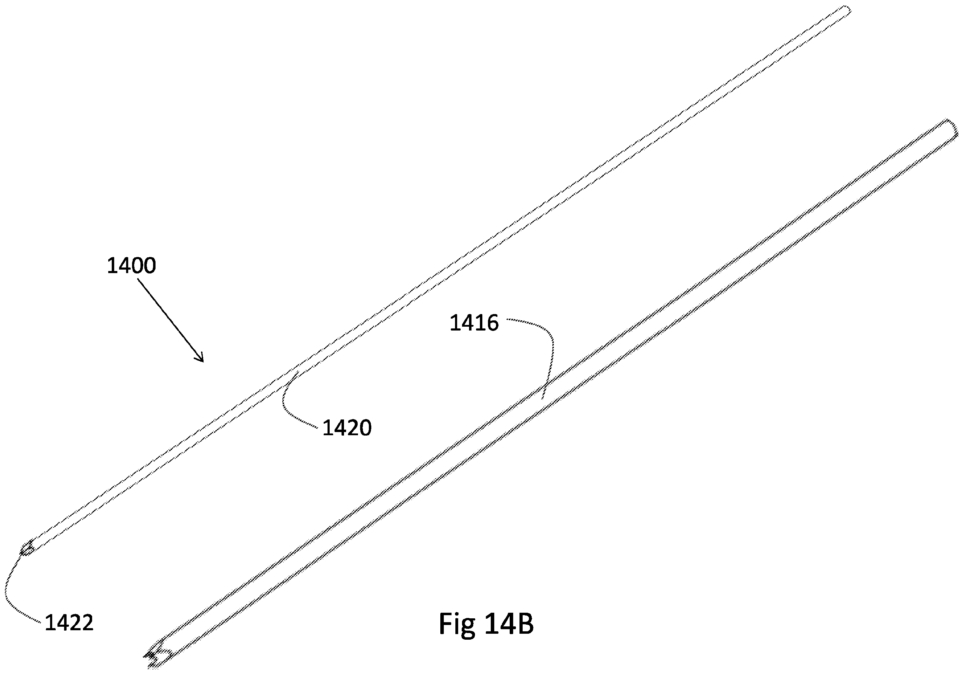

Reference is now made to figures 14A-14C which are schematic diagrams of an introducer assembly according to some embodiments of the present invention. Figure 14A shows a two part introducer assembly 1400 comprising an outer sleeve 1416 sharpened at the distal end 1406 which encloses an inner shaft 1420 sharpened at its distal end 1422. A gap of less than 10 mm, more preferably less than 5mm, and most preferably less than 3mm exists between the end of Inner shaft 1420 and the beginning of the cutting slope on sleeve 1416. The exposed tip 1422 of shaft 1420 enables anchoring of the shaft tip 1422 into the bone independent of the sleeve 1416. The sleeve 1416 and shaft 1420 are attached at or proximal to join point 1418 by for example welding or crimping. Join point 1418 is at the proximal side of the introducer assembly 1400 only. Once the assembly 1400 is inserted into the desired location in the subject, the join point 1418 is cut. Point 1418 optionally comprises a narrowing in outer sleeve 1416 as shown to facilitate and mark the cut. After cutting the sleeve is retracted. The separated sleeve 1416 and shaft 1420 are shown in figure 14B. It should be appreciated that shaft 1420 would be embedded in the desired position for fixture before sleeve 1416 is removed.

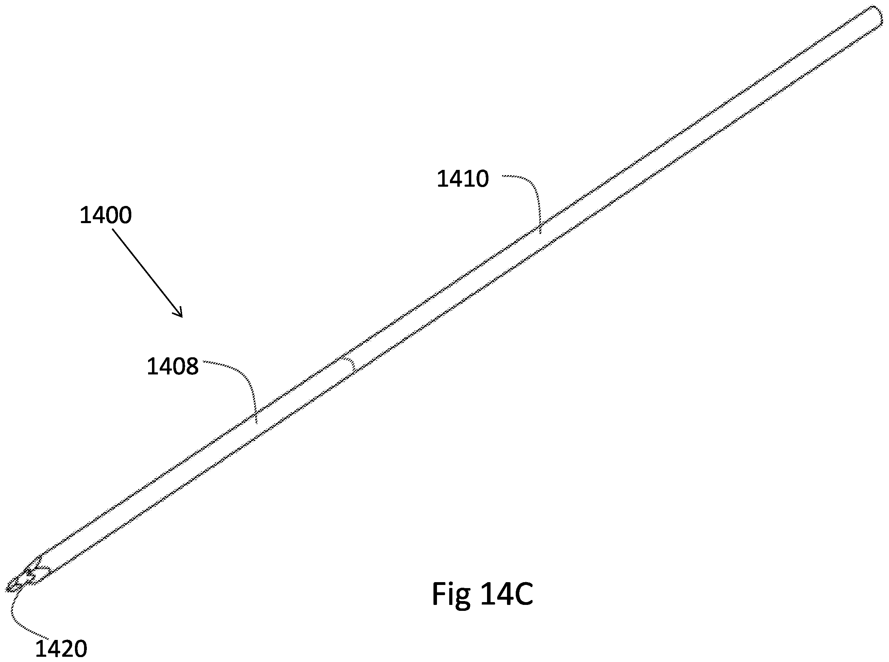

As shown in figure 14C, in a next step, implant 1408 is inserted on top of shaft 1420 which acts as a guide, and keeps the bones in place throughout the entire fixation procedure. Holding sleeve or instrument 1410 is also used to insert the implant 1408 onto shaft 1420 and to hold it is place so that inner shaft 1420 can be removed once implant 1408 is in position.

Preferably the outer diameter of the sleeve 1416 is smaller than the outer diameter of the implant 1408 by a factor of between 0.5%-10% resulting in better fixation of the implant and increasing final pull out strength. Preferably the implant has a pull out strength from bone of more than 2N, preferably more than 20N, preferably more than 60 N and most preferably more than 100 N.

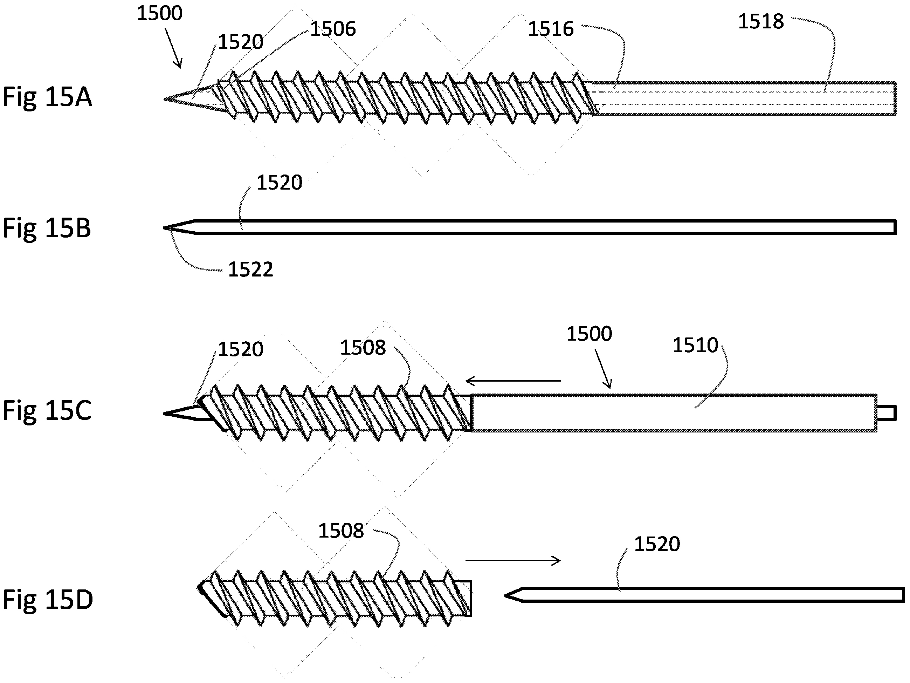

Reference is now made to figures 15A-15D which are schematic diagrams of an introducer assembly according to some embodiments of the present invention. Figure 15A shows a two part introducer assembly 1500 comprising an outer sleeve 1516 sharpened at the distal end 1506. Sleeve 1516 is threaded on its distal side allowing it to be screwed into the bone. Sleeve 1516 is not threaded on its proximal side allowing it to be held or otherwise manipulated. Sleeve 1516 encloses an inner shaft 1520 sharpened at its distal end 1522. The sleeve 1516 and shaft 1520 are attached at join point 1518 by for example welding or crimping. Join point 1518 is at the proximal side of the introducer assembly 1500 only. Once the assembly 1500 is inserted and screwed into the desired location in the subject, the join point 1518 is cut. It should be appreciated that shaft 1520 would be embedded in the desired position for fixture before sleeve 1516 is removed. Figure 15B shows shaft 1520 with sleeve 1516 removed.

As shown in figure 15C, in a next step, implant 1508 is inserted on top of shaft 1520 which acts as a guide, and keeps the bones in place throughout the entire fixation procedure. The thread created by sleeve 1516 is now used to guide the similarly threaded implant 1508 into position. Holding sleeve 1510 is also inserted onto shaft 1520 behind implant 1508 to help with insertion of implant 1508 and to hold it is place so that inner shaft 1520 can be removed (as shown in figure 15D) once implant 1508 is in position.

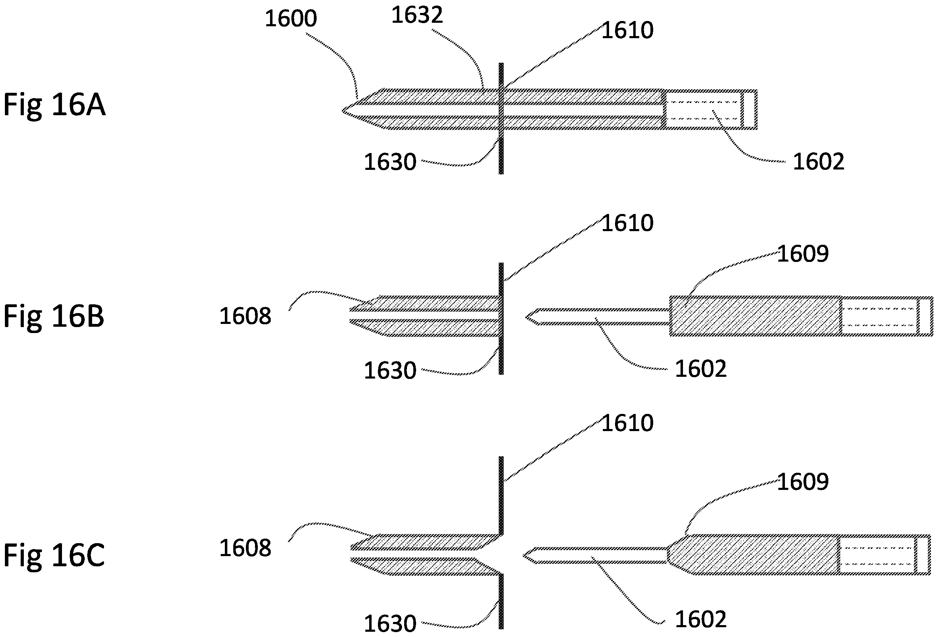

Reference is now made to figures 16A-16C which are schematic diagrams of an introducer assembly with an implant cut to size once positioned according to some embodiments of the present invention. As shown in figure 16A, an introducer assembly 1600 is positioned with a portion 1632 below the bone surface 1630. Assembly 1600 may be any embodiment of assembly as described herein. The implant 1608 may now be cut at the intersection point 1610 following removal of the introducer 1602 or alternatively before removal of the introducer 1602. The cut may be made for example with bone cutting forceps/scissors/reciprocating saw or hot wire to the desired length. Optionally, the implant 1608 may be pushed in 1-2 mm after cutting to avoid soft tissue irritation.

As shown in figures 16B and 16C, the implant 1608 is cut and the unneeded part of implant 1609 is removed along with introducer 1602. The difference between the cut of implant 1608 in figures 16B and 16C is that figure 16B shows a straight cut of implant 1608 and figure 16C shows a conical cut of implant 1608. Any other known cutting geometry may be used and the illustrations of figure 16 should not be considered limiting.

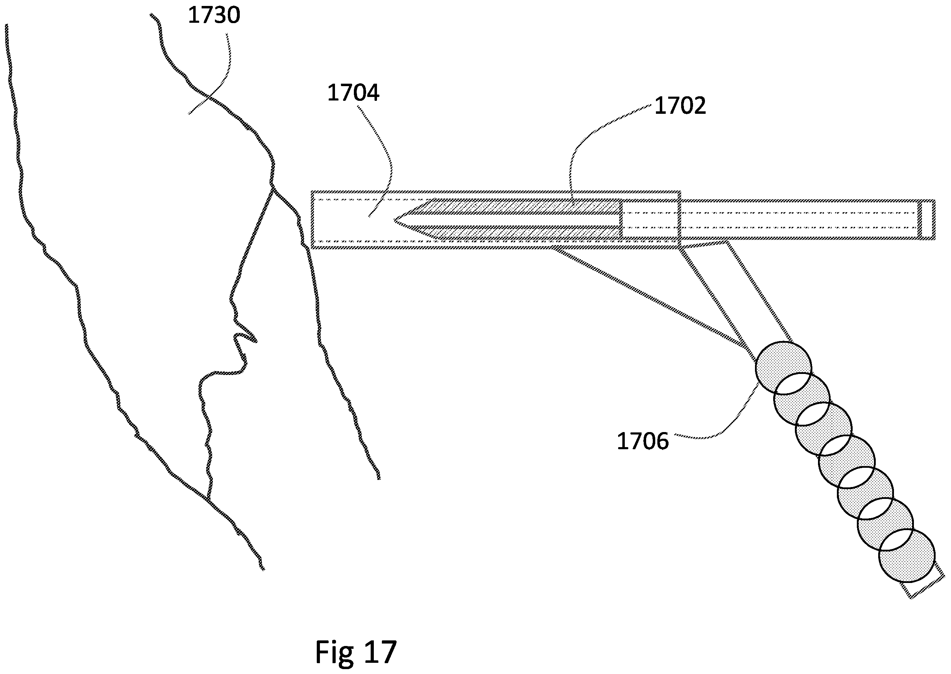

Reference is now made to figures 17 which is a schematic diagram of an introducer assembly positioning device according to some embodiments of the present invention. As shown in figure 17, introducer assembly 1702 which may be any embodiment of introducer assembly as described herein is held within positioning device 1704 which is held such as via handle 1706 and positioned over the bone 1730 of a subject. Assembly 1702 may then be driven into bone 1730 as described above. Once assembly 1702 is in position, device 1704 is removed, and introducer may be removed and the implant cut as described further above.

Other non-limiting, optional embodiments of introduction assemblies may include:
An introduction assembly with an introducer having a sharp penetrating tip wherein the tip includes elements that form a total diameter larger than the diameter of the shaft of the introducer but a diameter smaller than or equal to the outer diameter of the implant (similar to figure 12A). When the introducer is pushed into bone, these elements serve to create a hole/cavity. When the introducer is retracted through the implant, the elements bend or fold back such that introducer can be retracted through the inner cavity of the implant.

In an alternative embodiment, the introducer assembly comprises a sharp tip with a spiral geometry such that when the spiral is compressed a sharp tip, capable of bearing load is formed. When tensile force is applied to the spiral geometry tip, the spiral separates and can pass through a diameter smaller than the outer diameter of the sharp tip when it is under compression - such as being retracted through the implant.

Optionally, the distal segment of the introducer comprises components that partially dissociate upon retraction through the implant.

In an alternative embodiment, the inner shaft of the introducer or the sharp tip are themselves hollow and can collapse when subjected to force, such as during retraction of the tip through the inner diameter of the implant.

It should be appreciated that the above described methods and apparatus may be varied in many ways, including omitting or adding steps, changing the order of steps and the type of devices used. It should be appreciated that different features may be combined in different ways. In particular, not all the features shown above in a particular embodiment are necessary in every embodiment of the invention. Further combinations of the above features are also considered to be within the scope of some embodiments of the invention.

While the invention has been described with respect to a limited number of embodiments, it will be appreciated that many variations, modifications and other applications of the invention may be made.

## Claims

1. An introducer assembly (600) for introducing an orthopedic implant (608) into a subject comprising:
a. an introducer (602); and
b. said implant (608), wherein said implant (608) comprises a biocomposite material; wherein said introducer (602) initiates a cavity for said implant, wherein said assembly is inserted into said cavity to thereby extend said cavity, and wherein said introducer (602) is retracted from said assembly (600) leaving said implant (608) in said cavity;
wherein said biocomposite material comprises a biocompatible absorbable polymer, reinforcement filler and a coupling agent;
wherein said reinforcement filler comprises a volume of said implant (608) selected from the group consisting of: more than 40% w/w; and more than 45% w/w;
wherein the elastic modulus of said biocomposite material is selected from the group consisting of: 10 GPa, above 15 GPa, and above 20 GPa;
wherein the elastic modulus of said biocomposite material is selected from the group consisting of: not exceeding 100 GPa; and not exceeding 60 GPa; and
wherein the flexural modulus of said implant (608) is selected from the group consisting of: more than 200 MPa; more than 300 MPa; and more than 400 MPa;
**characterized in that** said introducer (602) can bear an axial force of more than 50N.

2. The assembly (600) of claim 1, wherein the structural strength of said biocomposite material contributes to the structural strength of said assembly

3. The assembly (600) of claim 1, wherein said introducer (602) comprises a material selected from the group consisting of:
a. 316 L stainless steel;
b. 316 LVM stainless steel;
c. 304 stainless steel;
d. titanium;
e. nitinol;
f. a biocompatible metal;
g. ceramic; and
h. a combination of the above

4. The assembly (600) of claim 1, wherein said introducer (602) has a hardness selected from the group consisting of:
a. 490 MPa (50 HV);
b. 981 MPa (100 HV); and;
c. 1961 MPa (200 HV).

5. The assembly (600) of claim 1, wherein the distal end of said introducer (602) is a sharp penetrative tip (606) and wherein said tip (606) can penetrate bone under compressive force or drilling.

6. The assembly (600) of claim 5, wherein said tip (606) comprises a plurality of tip parts (622) and said tip parts (622) each comprise an extraction rod (618) extending along a core (620) to the proximal end of said assembly; wherein said tip parts (622) are extracted following extraction of said core (620) by pulling said extraction rods (618); wherein extraction of said tip parts (622) is performed by a mechanism selected from the group consisting of: linear motion; rotation motion; compression; compression release; tensioning; tension release; screws; springs; and any combination of the above.

7. The assembly (600) of claim 1, wherein said introducer (602) can bear between 1.7 - 14.1 N^{∗}m (15-125 in-lbs) of torque.

8. The assembly (600) of claim 1, wherein said biocomposite material is bioabsorbable

9. The assembly (600) of claim 1, wherein said polymer is selected from the group consisting of: natural polymer, synthetic polymer, homo-polymer, co-polymer,
terpolymer, polyesters, aliphatic polyesters, polyorthoesters, polyanhydrides, polycarbonates, polyurethanes, polyamides, polyalky lene oxides, polylactides (PLA), poly-L-lactide (PLLA), poly-DL-lactide (PDLLA); polyglycolide (PGA); copolymers of glycolide; glycolide/trimethylene carbonate copolymers; (PGA/TMC); and other polymers from the PLA family.

10. The assembly (600) of claim 1, wherein said reinforcement filler is selected from the group consisting of: an organic material, an inorganic material; a mineral; a mineral chosen to increase the bioactivity of the implant; a mineral chosen to increase the mechanical strength of the implant; fibers; continuous fibers; a biodegradable glass, a cellulosic material, calcium phosphate, a nano-diamond, and a combination of any of the above.

11. The assembly (600) of claim 1, wherein said assembly further comprises a release mechanism to allow separation of said introducer and said implant, wherein said release mechanism is selected from the list consisting of: cutting a distally welded or crimped area to release internal locking; leavers; a ratchet mechanism; and a combination of the above.

12. The assembly (600) of claim 1, wherein said implant is formed as one of a: pin; wire, nail, screw, staple, plate; anchor; intramedullary nail; joint implant; spine implant, device for fracture fixation, device for tendon reattachment, device for spinal fixation, or a spinal cage; wherein said screw is selected from the group consisting of: cannulated; threaded; not threaded; partially threaded; self-tapping; self-drilling; and a combination of the above.

## Patentansprüche

1. Einführungsanordnung (600) zum Einführen eines orthopädischen Implantats (608) in einen Patienten, umfassend:
a. eine Einführvorrichtung (602); und
b. das Implantat (608), wobei das Implantat (608) ein Bioverbundstoffmaterial umfasst, wobei die Einführvorrichtung (602) einen Hohlraum für das Implantat initiiert, wobei die Anordnung in den Hohlraum eingeführt wird, um den Hohlraum dadurch zu erweitern, und wobei die Einführvorrichtung (602) aus der Anordnung (600) zurückgezogen wird, wobei das Implantat (608) in dem Hohlraum gelassen wird;
wobei das Bioverbundstoffmaterial ein biokompatibles absorbierbares Polymer, Verstärkungsfüllung und ein Kopplungsmittel umfasst;
wobei die Verstärkungsfüllung ein Volumen des Implantats (608) umfasst, das aus der Gruppe ausgewählt ist, die aus Folgendem besteht: mehr als 40 % w/w; und mehr als 45 % w/w;
wobei der Elastizitätsmodul des Bioverbundstoffmaterials aus der Gruppe ausgewählt ist, die aus Folgendem besteht: 10 GPa, mehr als 15 GPa und mehr als 20 GPa;
wobei der Elastizitätsmodul des Bioverbundstoffmaterials aus der Gruppe ausgewählt ist, die aus Folgendem besteht: 100 GPa nicht übersteigend; und 60 GPa nicht übersteigend; und
wobei der Biegemodul des Implantats (608) aus der Gruppe ausgewählt ist, die aus Folgendem besteht: mehr als 200 MPa; mehr als 300 MPa; und mehr als 400 MPa;
**dadurch gekennzeichnet, dass** die Einführvorrichtung (602) eine axiale Kraft von mehr als 50 N aushalten kann.

2. Anordnung (600) nach Anspruch 1, wobei die strukturelle Stärke des Bioverbundstoffmaterials zu der strukturellen Stärke der Anordnung beiträgt.

3. Anordnung (600) nach Anspruch 1, wobei die Einführvorrichtung (602) ein Material umfasst, das aus der Gruppe ausgewählt ist, die aus Folgendem besteht:
a. Edelstahl 316 L;
b. Edelstahl 316 LVM;
c. Edelstahl 304;
d. Titan;
e. Nitinol;
f. einem biokompatiblen Metall;
g. Keramik; und
h. einer Kombination des Vorstehenden.

4. Anordnung (600) nach Anspruch 1, wobei die Einführvorrichtung (602) eine Härte aufweist, die aus der Gruppe ausgewählt ist, die aus Folgendem besteht:
a. 490 MPa (50 HV);
b. 981 MPa (100 HV); und
c. 1961 MPa (200 HV).

5. Anordnung (600) nach Anspruch 1, wobei das distale Ende der Einführvorrichtung (602) eine scharfe penetrative Spitze (606) ist und wobei die Spitze (606) Knochen unter Kompressionskraft oder Bohren penetrieren kann.

6. Anordnung (600) nach Anspruch 5, wobei die Spitze (606) eine Vielzahl von Spitzenteilen (622) umfasst und die Spitzenteile (622) jeweils einen Extraktionsstab (618) umfassen, der sich entlang eines Kerns (620) zu dem proximalen Ende der Anordnung erstreckt; wobei die Spitzenteile (622) im Anschluss an die Extraktion des Kerns (620) extrahiert werden, indem die Extraktionsstäbe (618) gezogen werden; wobei Extraktion der Spitzenteile (622) durch einen Mechanismus durchgeführt wird, der aus der Gruppe ausgewählt ist, die aus Folgendem besteht: linearer Bewegung; Drehbewegung, Kompression; Kompressionsfreigabe; Spannung; Spannungsfreigabe; Schrauben; Federn und einer beliebigen Kombination des Vorstehenden.

7. Anordnung (600) nach Anspruch 1, wobei die Einführvorrichtung (602) zwischen 1,7-14,1 N*m (15-125 in-lbs) Drehmoment aushalten kann.

8. Anordnung (600) nach Anspruch 1, wobei das Bioverbundstoffmaterial bioabsorbierbar ist.

9. Anordnung (600) nach Anspruch 1, wobei das Polymer aus der Gruppe ausgewählt ist, die aus Folgendem besteht: natürlichem Polymer, synthetischem Polymer, Homopolymer, Copolymer, Terpolymer, Polyestern, aliphatischen Polyestern, Polyorthoestern, Polyanhydriden, Polycarbonaten, Polyurethanen, Polyamiden, Polyalkylenoxiden, Polylactiden (PLA), Poly-L-Lactid (PLLA), Poly-DL-Lactid (PDLLA); Polyglycolid (PGA); Copolymeren von Glycolid; Glycolid/Trimethylencarbonat-Copolymeren; (PGA/TMC) und anderen Polymeren der PLA-Familie.

10. Anordnung (600) nach Anspruch 1, wobei die Verstärkungsfüllung aus der Gruppe ausgewählt ist, die aus Folgendem besteht: einem organischen Material, einem anorganischen Material; einem Mineral; einem Mineral, das derart ausgewählt ist, dass es die Bioaktivität des Implantats erhöht; einem Mineral, das derart ausgewählt ist, dass es die mechanische Stärke des Implantats erhöht, Fasern; durchgehenden Fasern; einem biologisch abbaubaren Glas, einem Cellulosematerial, Calciumphosphat, einem Nanodiamanten und einer Kombination aus beliebigen des Vorstehenden.

11. Anordnung (600) nach Anspruch 1, wobei die Anordnung ferner einen Freigabemechanismus umfasst, um die Trennung der Einführvorrichtung und des Implantats zu ermöglichen, wobei der Freigabemechanismus aus der Liste ausgewählt ist, die aus Folgendem besteht: Schneiden eines distal geschweißten oder gecrimpten Bereichs, um interne Verriegelung freizugeben; Hebeln; einem Sperrklinkenmechanismus und einer Kombination des Vorstehenden.

12. Anordnung (600) nach Anspruch 1, wobei das Implantat als eines des Folgenden gebildet ist: Stift, Draht, Nagel, Schraube, Heftklammer, Platte; Anker; intramedullärer Nagel; Gelenkimplantat; Wirbelimplantat, Vorrichtung zur Frakturfixierung, Vorrichtung zur Sehnenneuanbringung, Vorrichtung zur Wirbelfixierung oder Wirbelplatzhalter; wobei die Schraube aus der Gruppe ausgewählt ist, die aus Folgendem besteht: kanüliert; gewunden; nicht gewunden; teilweise gewunden; selbstschneidend; selbstbohrend und einer Kombination des Vorstehenden.

## Revendications

1. Bloc introducteur (600) pour introduire un implant orthopédique (608) dans un sujet comprenant :
a. un introducteur (602) ; et
b. ledit implant (608), dans lequel ledit implant (608) comprend un matériau biocomposite ; dans lequel ledit introducteur (602) initie une cavité pour ledit implant, dans lequel ledit bloc est inséré dans ladite cavité pour ainsi étendre ladite cavité, et dans lequel ledit introducteur (602) est rétracté à partir dudit bloc (600) en laissant ledit implant (608) dans ladite cavité ;
dans lequel ledit matériau biocomposite comprend un polymère absorbable biocompatible, une charge de renforcement et un agent de couplage ;
dans lequel ladite charge de renforcement comprend un volume dudit implant (608) choisi dans le groupe constitué par : plus de 40 % p/p ; et plus de 45 % p/p ;
dans lequel le module élastique dudit matériau biocomposite est choisi dans le groupe constitué par : 10 GPa, au-dessus de 15 GPa et au-dessus de 20 GPa ;
dans lequel le module élastique dudit matériau biocomposite est choisi dans le groupe constitué par : ne dépassant pas 100 GPa ; et ne dépassant pas 60 GPa ; et dans lequel le module de résistance à la flexion dudit implant (608) est choisi dans le groupe constitué par : plus de 200 MPa ; plus de 300 MPa ; et plus de 400 MPa ; **caractérisé en ce que** ledit introducteur (602) peut supporter une force axiale de plus de 50 N.

2. Bloc (600) selon la revendication 1, dans lequel la résistance structurelle dudit matériau biocomposite contribue à la résistance structurelle dudit bloc.

3. Bloc (600) selon la revendication 1, dans lequel ledit introducteur (602) comprend un matériau choisi dans le groupe constitué par :
a. l'acier inoxydable 316 L ;
b. l'acier inoxydable 316 LVM ;
c. l'acier inoxydable 304 ;
d. le titane ;
e. le nitinol ;
f. un métal biocompatible ;
g. la céramique ; et
h. une combinaison de ce qui précède.

4. Bloc (600) selon la revendication 1, dans lequel ledit introducteur (602) a une dureté choisie dans le groupe constitué par :
a. 490 MPa (50 HV) ;
b. 981 MPa (100 HV) ; et ;
c. 1 961 MPa (200 HV).

5. Bloc (600) selon la revendication 1, dans lequel l'extrémité distale dudit introducteur (602) est une pointe pénétrante acérée (606) et dans lequel ladite pointe (606) peut pénétrer dans un os sous l'effet d'une force de compression ou d'un perçage.

6. Bloc (600) selon la revendication 5, dans lequel ladite pointe (606) comprend une pluralité de parties de pointe (622) et lesdites parties de pointe (622) comprennent chacune une tige d'extraction (618) s'étendant le long d'une âme (620) vers l'extrémité proximale dudit bloc ; dans lequel lesdites parties de pointe (622) sont extraites à la suite de l'extraction de ladite âme (620) en tirant lesdites tiges d'extraction (618) ; dans lequel l'extraction desdites parties de pointe (622) est menée à bien par un mécanisme choisi dans le groupe constitué par : un mouvement linéaire ; un mouvement de rotation ; une compression ; un relâchement de compression ; une mise en tension ; un relâchement de tension ; des vis ; des ressorts ; et une quelconque combinaison de ce qui précède.

7. Bloc (600) selon la revendication 1, dans lequel ledit introducteur (602) peut supporter entre 1,7 et 14,1 N*m (15 à 125 pouces-livre) de couple.

8. Bloc (600) selon la revendication 1, dans lequel ledit matériau biocomposite est biorésorbable.

9. Bloc (600) selon la revendication 1, dans lequel ledit polymère est choisi dans le groupe constitué par : un polymère naturel, un polymère synthétique, un homopolymère, un copolymère, un terpolymère, des polyesters, des polyesters aliphatiques, des polyorthoesters, des polyanhydrides, des polycarbonates, des polyuréthanes, des polyamides, des polyoxydes d'alkylène, des polylactides (PLA), un poly-L-lactide (PLLA), un poly-DL-lactide (PDLLA) ; un polyglycolide (PGA) ; des copolymères de glycolide ; des copolymères de glycolide/carbonate de triméthylène ; (PGA/TMC) ; et d'autres polymères issus de la famille PLA.

10. Bloc (600) selon la revendication 1, dans lequel ladite charge de renforcement est choisie dans le groupe constitué par : un matériau organique, un matériau inorganique ; un minéral ; un minéral choisi pour accroître la bioactivité de l'implant ; un minéral choisi pour accroître la résistance mécanique de l'implant ; des fibres ; des fibres continues ; un verre biodégradable, un matériau cellulosique, du phosphate de calcium, un nano-diamant et une combinaison de l'un quelconque de ce qui précède.

11. Bloc (600) selon la revendication 1, dans lequel ledit bloc comprend en outre un mécanisme de relâchement pour permettre la séparation dudit introducteur et dudit implant, dans lequel ledit mécanisme de relâchement est choisi dans la liste constituée par : la découpe d'une zone soudée ou sertie de manière distale pour défaire un verrouillage interne ; des éléments amovibles ; un mécanisme de cliquet ; et une combinaison de ce qui précède.

12. Bloc (600) selon la revendication 1, dans lequel ledit implant est formé en tant que l'un parmi : une broche ; un fil, un clou, une vis, une agrafe, une plaque ; une ancre ; un clou intramédullaire ; un implant d'articulation ; un implant vertébral, un dispositif pour la fixation de fractures, un dispositif pour la réimplantation de tendons, un dispositif pour la fixation vertébrale ou une cage vertébrale ; dans lequel ladite vis est choisie dans le groupe constitué par : canulé ; fileté ; non fileté ; partiellement fileté ; auto-taraudant ; auto-perçant ; et une combinaison de ce qui précède.
